# EUROPEAN PATENT SPECIFICATION

(11) **EP 1 434 993 B1**
(45) Date of publication and mention of the grant of the patent: **24.04.2013**
(21) Application number: 02761695.2
(22) Date of filing: 18.09.2002
(51) Int. Cl.: G01N 33/74, G01N 33/68

(54) **METHODS OF ASSAYING CONNECTIVE TISSUE GROWTH FACTOR**
VERFAHREN ZUM TESTEN DES BINDEGEWEBE-WACHSTUMSFAKTORS
PROCEDES PERMETTANT D'ANALYSER LE FACTEUR DE CROISSANCE DU TISSU CONJONCTIF

(30) Priority: 18.09.2001 US 323305 P
(43) Date of publication of application: 07.07.2004
(73) Proprietor: Fibrogen, Inc., San Francisco, CA 94158 (US)
(72) Inventor: WEITZ, Stephen, L., Oakland, CA 94619 (US); USINGER, William, R., Lafayatte, CA 94549 (US)
(74) Representative: Goodfellow, Hugh Robin
(86) International application number: PCT/US2002/029500
(87) International publication number: WO 2003/024308

(56) References cited:
- EP-A1- 1 043 335
- WO-A-00/35936
- WO-A2-00/35939
- US-A- 5 408 040
- US-A- 5 420 016
- US-A- 5 876 730
- US-A- 5 916 756
- US-A- 6 149 916
- TAMATANI TAKUYA ET AL: "Establishment of the enzyme-linked immunosorbent assay for connective tissue growth factor (CTGF) and its detection in the sera of biliary atresia" BIOCHEMICAL AND BIOPHYSICAL RESEARCH COMMUNICATIONS, vol. 25, no. 3, 29 October 1998 (1998-10-29), pages 748-752, XP002961806 ISSN: 0006-291X
- YANG DOO-HYUN ET AL: "Identification of glycosylated 38-kDa connective tissue growth factor (IGFBP-related protein 2) and proteolytic fragments in human biological fluids, and up-regulation of IGFBP-rP2 expression by TGF-beta in Hs578T human breast cancer cells" JOURNAL OF CLINICAL ENDOCRINOLOGY AND METABOLISM, vol. 83, no. 7, July 1998 (1998-07), pages 2593-2596, XP002403407 ISSN: 0021-972X
- RISER BRUCE L ET AL: "Regulation of connective tissue growth factor activity in cultured rat mesangial cells and its expression in experimental diabetic glomerulosclerosis" JOURNAL OF THE AMERICAN SOCIETY OF NEPHROLOGY, vol. 11, no. 1, January 2000 (2000-01), pages 25-38, XP002403408 ISSN: 1046-6673
- BALL DEANNA K ET AL: "Characterization of 16- to 20-kilodalton (kDa) connective tissue growth factors (CTGFs) and demonstration of proteolytic activity for 38-kDa CTGF in pig uterine luminal flushings" BIOLOGY OF REPRODUCTION, vol. 59, no. 4, October 1998 (1998-10), pages 828-835, XP002403409 ISSN: 0006-3363
- KOHLER ET AL.: 'Continuous cultures of fused cells secreting antibody of predefined specificity' NATURE vol. 256, 07 August 1975, pages 495 - 497, XP002044294
- TAMATANI ET AL.: 'Establishment of the enzyme-linked immunosorbent assay for connective tissue growth factor (CTGF) and its detection in the sera of biliary atresia' BIOCHEMICAL AND BIOPHYSICAL RESEARCH COMMUNICATIONS vol. 251, 1998, pages 748 - 752, XP002961806

### SEQUENCE LISTING

<110> Weitz, Stephen L Usinger, William R
<120> METHODS OF ASSAYING CONNECTIVE TISSUE GROWTH FACTOR
<130> FP0812 PCT
<150> US 60/323,305
   <151> 2001-09-18
<160> 8
<170> PatentIn version 3.1
<210> 1
   <211> 1050
   <212> DNA
   <213> Homo sapiens
<400> 1
   atgaccgccg ccagtatggg ccccgtccgc gtcgccttcg tggtcctcct cgccctctgc     60
   agccggccgg ccgtcggcca gaactgcagc gggccgtgcc ggtgcccgga cgagccggcg    120
   ccgcgctgcc cggcgggcgt gagcctcgtg ctggacggct gcggctgctg ccgcgtctgc    180
   gccaagcagc tgggcgagct gtgcaccgag cgcgacccct gcgacccgca caagggcctc    240
   ttctgtgact tcggctcccc ggccaaccgc aagatcggcg tgtgcaccgc caaagatggt    300
   gctccctgca tcttcggtgg tacggtgtac cgcagcggag agtccttcca gagcagctgc    360
   aagtaccagt gcacgtgcct ggacggggcg gtgggctgca tgcccctgtg cagcatggac    420
   gttcgtctgc ccagccctga ctgccccttc ccgaggaggg tcaagctgcc cgggaaatgc    480
   tgcgaggagt gggtgtgtga cgagcccaag gaccaaaccg tggttgggcc tgccctcgcg    540
   gcttaccgac tggaagacac gtttggccca gacccaacta tgattagagc caactgcctg    600
   gtccagacca cagagtggag cgcctgttcc aagacctgtg ggatgggcat ctccacccgg    660
   gttaccaatg acaacgcctc ctgcaggcta gagaagcaga gccgcctgtg catggtcagg    720
   ccttgcgaag ctgacctgga agagaacatt aagaagggca aaaagtgcat ccgtactccc    780
   aaaatctcca agcctatcaa gtttgagctt tctggctgca ccagcatgaa gacataccga    840
   gctaaattct gtggagtatg taccgacggc cgatgctgca ccccccacag aaccaccacc    900
   ctgccggtgg agttcaagtg ccctgacggc gaggtcatga agaagaacat gatgttcatc    960
   aagacctgtg cctgccatta caactgtccc ggagacaatg acatctttga atcgctgtac   1020
   tacaggaaga tgtacggaga catggcatga                                    1050
<210> 2
   <211> 349
   <212> PRT
   <213> Homo sapiens
<400> 2
   Met Thr Ala Ala Ser Met Gly Pro Val Arg Val Ala Phe Val Val Leu
   1               5                   10                  15
   Leu Ala Leu Cys Ser Arg Pro Ala Val Gly Gln Asn Cys Ser Gly Pro
               20                  25                  30
   Cys Arg Cys Pro Asp Glu Pro Ala Pro Arg Cys Pro Ala Gly Val Ser
           35                  40                  45
   Leu Val Leu Asp Gly Cys Gly Cys Cys Arg Val Cys Ala Lys Gln Leu
       50                  55                  60
   Gly Glu Leu Cys Thr Glu Arg Asp Pro Cys Asp Pro His Lys Gly Leu
   65                  70                  75                  80
   Phe Cys Asp Phe Gly Ser Pro Ala Asn Arg Lys Ile Gly Val Cys Thr
                   85                  90                  95
   Ala Lys Asp Gly Ala Pro Cys Ile Phe Gly Gly Thr Val Tyr Arg Ser
               100                 105                 110
   Gly Glu Ser Phe Gln Ser Ser Cys Lys Tyr Gln Cys Thr Cys Leu Asp
           115                 120                 125
   Gly Ala Val Gly Cys Met Pro Leu Cys Ser Met Asp Val Arg Leu Pro
       130                 135                 140
   Ser Pro Asp Cys Pro Phe Pro Arg Arg Val Lys Leu Pro Gly Lys Cys
   145                 150                 155                 160
   Cys Glu Glu Trp Val Cys Asp Glu Pro Lys Asp Gln Thr Val Val Gly
                   165                 170                 175
   Pro Ala Leu Ala Ala Tyr Arg Leu Glu Asp Thr Phe Gly Pro Asp Pro
               180                 185                 190
   Thr Met Ile Arg Ala Asn Cys Leu Val Gln Thr Thr Glu Trp Ser Ala
           195                 200                 205
   Cys Ser Lys Thr Cys Gly Met Gly Ile Ser Thr Arg Val Thr Asn Asp
       210                 215                 220
   Asn Ala Ser Cys Arg Leu Glu Lys Gln Ser Arg Leu Cys Met Val Arg
   225 230 235 240
   Pro Cys Glu Ala Asp Leu Glu Glu Asn Ile Lys Lys Gly Lys Lys Cys
   245 250 255
   Ile Arg Thr Pro Lys Ile Ser Lys Pro Ile Lys Phe Glu Leu Ser Gly
   260 265 270
   Cys Thr Ser Met Lys Thr Tyr Arg Ala Lys Phe Cys Gly Val Cys Thr
   275 280 285
   Asp Gly Arg Cys Cys Thr Pro His Arg Thr Thr Thr Leu Pro Val Glu
   290 295 300
   Phe Lys Cys Pro Asp Gly Glu Val Met Lys Lys Asn Met Met Phe Ile
   305 310 315 320
   Lys Thr Cys Ala Cys His Tyr Asn Cys Pro Gly Asp Asn Asp Ile Phe
   325 330 335
   Glu Ser Leu Tyr Tyr Arg Lys Met Tyr Gly Asp Met Ala
   340 345
<210> 3
   <211> 33
   <212> DNA
   <213> Homo sapiens
<400> 3
   gctccgcccg cagtgggatc catgaccgcc gcc 33
<210> 4
   <211> 30
   <212> DNA
   <213> Homo sapiens
<400> 4
   ggatccggat cctcatgcca tgtctccgta 30
<210> 5
   <211> 349
   <212> PRT
   <213> Bos taurus
<400> 5
   Met Ser Ala Thr Gly Leu Gly Pro Val Arg Cys Ala Phe Val Leu Leu
   1 5 10 15
   Leu Ala Leu Cys Ser Arg Pro Ala Ser Ser Gln Asp Cys Ser Ala Pro
               20                  25                  30
   Cys Gln Cys Pro Ala Gly Pro Ala Pro Arg Cys Pro Ala Gly Val Ser
           35                  40                  45
   Leu Val Leu Asp Gly Cys Gly Cys Cys Arg Val Cys Ala Lys Gln Leu
       50                  55                  60
   Ser Glu Leu Cys Thr Glu Arg Asp Pro Cys Asp Pro His Lys Gly Leu
   65                  70                  75                  80
   Phe Cys Asp Phe Gly Ser Pro Ala Asn Arg Lys Ile Gly Val Cys Thr
                   85                  90                  95
   Ala Lys Asp Gly Ala Pro Cys Val Phe Gly Gly Thr Val Tyr Gln Ser
               100                 105                 110
   Gly Glu Ser Phe Gln Ser Ser Cys Lys Tyr Gln Cys Thr Cys Leu Asp
           115                 120                 125
   Gly Ser Val Gly Cys Val Pro Leu Cys Ser Val Asp Val Arg Leu Pro
       130                 135                 140
   Ser Pro Asp Cys Pro Phe Pro Arg Arg Val Lys Leu Pro Gly Lys Cys
   145                 150                 155                 160
   Cys Glu Glu Trp Val Cys Asp Glu Pro Lys Glu His Thr Val Val Gly
                   165                 170                 175
   Pro Ala Leu Ala Ala Tyr Arg Pro Glu Asp Thr Phe Gly Pro Asp Pro
               180                 185                 190
   Thr Met Ile Arg Ala Asn Cys Leu Val Gln Thr Thr Glu Trp Ser Ala
           195                 200                 205
   Cys Ser Lys Thr Cys Gly Met Gly Ile Ser Thr Arg Val Thr Asn Asp
       210                 215                 220
   Asn Ala Phe Cys Arg Leu Glu Lys Gln Ser Arg Leu Cys Met Val Arg
   225                 230                 235                 240
   Pro Cys Glu Ala Asp Leu Glu Glu Asn Ile Lys Lys Gly Lys Lys Cys
                   245                 250                 255
   Ile Arg Thr Pro Lys Ile Ser Lys Pro Ile Lys Phe Glu Leu Ser Gly
   260 265 270
   Cys Thr Ser Met Lys Thr Tyr Arg Ala Lys Phe Cys Gly Val Cys Thr
   275 280 285
   Asp Gly Arg Cys Cys Thr Pro His Arg Thr Thr Thr Leu Pro Val Glu
   290 295 300
   Phe Lys Cys Pro Asp Gly Glu Val Met Lys Lys Ser Met Met Phe Ile
   305 310 315 320
   Lys Thr Cys Ala Cys His Tyr Asn Cys Pro Gly Asp Asn Asp Ile Phe
   325 330 335
   Glu Ser Leu Tyr Tyr Arg Lys Met Tyr Gly Asp Met Ala
   340 345
<210> 6
   <211> 349
   <212> PRT
   <213> Sus scrofa
<400> 6
   Met Ser Ala Thr Gly Leu Ser Pro Val Arg Cys Ala Phe Val Leu Leu
   1 5 10 15
   Leu Ala Leu Cys Ser Arg Pro Ala Ser Gly Gln Asp Cys Ser Gly Gln
   20 25 30
   Cys Gln Cys Ala Ala Gly Lys Arg Arg Ala Cys Pro Ala Gly Val Ser
   35 40 45
   Leu Val Leu Asp Gly Cys Gly Cys Cys Arg Leu Cys Ala Lys Gln Leu
   50 55 60
   Gly Glu Leu Cys Thr Glu Arg Asp Pro Cys Asp Pro His Lys Gly Leu
   65 70 75 80
   Phe Cys Asp Phe Gly Ser Pro Ala Asn Arg Lys Ile Gly Val Cys Thr
   85 90 95
   Ala Lys Asp Gly Ala Pro Cys Val Phe Gly Gly Thr Val Tyr Arg Ser
   100 105 110
   Gly Glu Ser Phe Gln Ser Ser Cys Lys Tyr Gln Cys Thr Cys Leu Asp
   115 120 125
   Gly Ala Val Gly Cys Val Pro Leu Cys Ser Met Asp Val Arg Leu Pro
       130                 135                 140
   Ser Pro Asp Cys Pro Phe Pro Arg Arg Val Lys Leu Pro Gly Lys Cys
   145                 150                 155                 160
   Cys Glu Glu Trp Val Cys Asp Glu Pro Lys Asp His Thr Val Val Gly
                   165                 170                 175
   Pro Ala Leu Ala Ala Tyr Arg Leu Glu Asp Thr Phe Gly Pro Asp Pro
               180                 185                 190
   Thr Met Met Arg Ala Asn Cys Leu Val Gln Thr Thr Glu Trp Ser Ala
           195                 200                 205
   Cys Ser Lys Thr Cys Gly Met Gly Ile Ser Thr Arg Val Thr Asn Asp
       210                 215                 220
   Asn Ala Phe Cys Arg Leu Glu Lys Gln Ser Arg Leu Cys Met Val Arg
   225                 230                 235                 240
   Pro Cys Glu Ala Asp Leu Glu Glu Asn Ile Lys Lys Gly Lys Lys Cys
                   245                 250                 255
   Ile Arg Thr Pro Lys Ile Ser Lys Pro Val Lys Phe Glu Leu Ser Gly
               260                 265                 270
   Cys Thr Ser Val Lys Thr Tyr Arg Ala Lys Phe Cys Gly Val Cys Thr
           275                 280                 285
   Asp Gly Arg Cys Cys Thr Pro His Arg Thr Thr Thr Leu Pro Val Glu
       290                 295                 300
   Phe Lys Cys Pro Asp Gly Glu Val Met Lys Lys Ser Met Met Phe Ile
   305                 310                 315                 320
   Lys Thr Cys Ala Cys His Tyr Asn Cys Pro Gly Asp Asn Asp Ile Phe
                   325                 330                 335
   Glu Ser Leu Tyr Tyr Arg Lys Met Tyr Gly Asp Met Ala
               340                 345
<210> 7
   <211> 347
   <212> PRT
   <213> Rattus norvegicus
<400> 7
   Met Leu Ala Ser Val Ala Gly Pro Val Ser Leu Ala Leu Val Leu Leu
   1               5                   10                  15
   Leu Cys Thr Arg Pro Ala Thr Gly Gln Asp Cys Ser Ala Gln Cys Gln
               20                  25                  30
   Cys Ala Ala Glu Ala Ala Pro Arg Cys Pro Ala Gly Val Ser Leu Val
           35                  40                  45
   Leu Asp Gly Cys Gly Cys Cys Arg Val Cys Ala Lys Gln Leu Gly Glu
       50                  55                  60
   Leu Cys Thr Glu Arg Asp Pro Cys Asp Pro His Lys Gly Leu Phe Cys
   65                  70                  75                  80
   Asp Phe Gly Ser Pro Ala Asn Arg Lys Ile Gly Val Cys Thr Ala Lys
                   85                  90                  95
   Asp Gly Ala Pro Cys Val Phe Gly Gly Ser Val Tyr Arg Ser Gly Glu
               100                 105                 110
   Ser Phe Gln Ser Ser Cys Lys Tyr Gln Cys Thr Cys Leu Asp Gly Ala
           115                 120                 125
   Val Gly Cys Val Pro Leu Cys Ser Met Asp Val Arg Leu Pro Ser Pro
       130                 135                 140
   Asp Cys Pro Phe Pro Arg Arg Val Lys Leu Pro Gly Lys Cys Cys Glu
   145                 150                 155                 160
   Glu Trp Val Cys Asp Glu Pro Lys Asp Arg Thr Val Val Gly Pro Ala
                   165                 170                 175
   Leu Ala Ala Tyr Arg Leu Glu Asp Thr Phe Gly Pro Asp Pro Thr Met
               180                 185                 190
   Met Arg Ala Asn Cys Leu Val Gln Thr Thr Glu Trp Ser Ala Cys Ser
           195                 200                 205
   Lys Thr Cys Gly Met Gly Ile Ser Thr Arg Val Thr Asn Asp Asn Thr
       210                 215                 220
   Phe Cys Arg Leu Glu Lys Gln Ser Arg Leu Cys Met Val Arg Pro Cys
   225                 230                 235                 240
   Glu Ala Asp Leu Glu Glu Asn Ile Lys Lys Gly Lys Lys Cys Ile Arg
                   245                     250                 255
   Thr Pro Lys Ile Ala Lys Pro Val Lys Phe Glu Leu Ser Gly Cys Thr
               260                 265                 270
   Ser Val Lys Thr Tyr Arg Ala Lys Phe Cys Gly Val Cys Thr Asp Gly
           275                 280                 285
   Arg Cys Cys Thr Pro His Arg Thr Thr Thr Leu Pro Val Glu Phe Lys
       290                 295                 300
   Cys Pro Asp Gly Glu Ile Met Lys Lys Asn Met Met Phe Ile Lys Thr
   305                 310                 315                     320
   Cys Ala Cys His Tyr Asn Cys Pro Gly Asp Asn Asp Ile Phe Glu Ser
                   325                 330                 335
   Leu Tyr Tyr Arg Lys Met Tyr Gly Asp Met Ala
               340                 345
<210> 8
   <211> 348
   <212> PRT
   <213> Mus musculus
<400> 8
   Met Leu Ala Ser Val Ala Gly Pro Ile Ser Leu Ala Leu Val Leu Leu
   1               5                   10                  15
   Ala Leu Cys Thr Arg Pro Ala Thr Gly Gln Asp Cys Ser Ala Gln Cys
               20                  25                  30
   Gln Cys Ala Ala Glu Ala Ala Pro His Cys Pro Ala Gly Val Ser Leu
           35                  40                  45
   Val Leu Asp Gly Cys Gly Cys Cys Arg Val Cys Ala Lys Gln Leu Gly
       50                  55                  60
   Glu Leu Cys Thr Glu Arg Asp Pro Cys Asp Pro His Lys Gly Leu Phe
   65                  70                  75                  80
   Cys Asp Phe Gly Ser Pro Ala Asn Arg Lys Ile Gly Val Cys Thr Ala
                   85                  90                  95
   Lys Asp Gly Ala Pro Cys Val Phe Gly Gly Ser Val Tyr Arg Ser Gly
               100                 105                 110
   Glu Ser Phe Gln Ser Ser Cys Lys Tyr Gln Cys Thr Cys Leu Asp Gly
           115                 120                 125
   Ala Val Gly Cys Val Pro Leu Cys Ser Met Asp Val Arg Leu Pro Ser
       130                 135                 140
   Pro Asp Cys Pro Phe Pro Arg Arg Val Lys Leu Pro Gly Lys Cys Cys
   145                 150                 155                 160
   Lys Glu Trp Val Cys Asp Glu Pro Lys Asp Arg Thr Ala Val Gly Pro
                   165                 170                 175
   Ala Leu Ala Ala Tyr Arg Leu Glu Asp Thr Phe Gly Pro Asp Pro Thr
               180                 185                 190
   Met Met Arg Ala Asn Cys Leu Val Gln Thr Thr Glu Trp Ser Ala Cys
           195                 200                 205
   Ser Lys Thr Cys Gly Met Gly Ile Ser Thr Arg Val Thr Asn Asp Asn
       210                 215                 220
   Thr Phe Cys Arg Leu Glu Lys Gln Ser Arg Leu Cys Met Val Arg Pro
   225                 230                 235                 240
   Cys Glu Ala Asp Leu Glu Glu Asn Ile Lys Lys Gly Lys Lys Cys Ile
                   245                 250                 255
   Arg Thr Pro Lys Ile Ala Lys Pro Val Lys Phe Glu Leu Ser Gly Cys
               260                 265                 270
   Thr Ser Val Lys Thr Tyr Arg Ala Lys Phe Cys Gly Val Cys Thr Asp
           275                 280                 285
   Gly Arg Cys Cys Thr Pro His Arg Thr Thr Thr Leu Pro Val Glu Phe
       290                 295                 300
   Lys Cys Pro Asp Gly Glu Ile Met Lys Lys Asn Met Met Phe Ile Lys
   305                 310                 315                 320
   Thr Cys Ala Cys His Tyr Asn Cys Pro Gly Asp Asn Asp Ile Phe Glu
                   325                 330                 335
   Ser Leu Tyr Tyr Arg Lys Met Tyr Gly Asp Met Ala
   340 345

## Description

### FIELD OF THE INVENTION

The present invention relates to detection and quantification of connective tissue growth factor (CTGF), and diagnosis and detection of various CTGF-associated diseases and disorders.

### BACKGROUND OF THE INVENTION

Connective tissue growth factor (CTGF) is a mitogenic, chemotactic, and extracellular matrix-inducing factor for fibroblasts and other connective tissue cells or other cells capable of producing extracellular matrix. CTGF polypeptides and gene sequences have been identified from a number of species including human. (See, e.g., Ryseck et al. (1991) Cell Growth Differ 2:225-233; International Publication No. WO 00/27868, published 18 May 2000; and Bradham et al. (1991) J Cell Biol 114:1285-1294.) Human CTGF is a 349-residue polypeptide encoded by an open reading frame of 1047 nucleotides, with an initiation site at about nucleotide 130 and a TGA termination site at about nucleotide 1177, relative to the full-length cDNA. (See, e.g., U.S. Patent No. 5,408,040; and GenBank Accession Number NP001892.) US Patent No. 5,916,756 describes CTGF, means for producing CTGF, as well as diagnostic and therapeutic methods for using CTGF.

Pathologically, CTGF is involved in conditions where there is an overgrowth of connective tissue cells and over-deposition of extracellular matrix, including such diseases as fibrosis and excess scarring of the skin and major organs, cancer, systemic sclerosis, angiogenesis, arteriosclerosis, atherosclerosis, diabetic nephropathy, and renal hypertension. (See, e.g., U.S. Patent No. 5,408,040; International Publication No. WO 01/15729, published 8 March 2001.) Additionally, fragments of CTGF have been associated with certain biological activities. (See, e.g., International Publication No. WO 00/35939, published 12 October 2000; International Publication No. WO 00/35936, published 22 June 2000; U.S. Patent No. 5,876,730; Brigstock et al. (1997) J Biol Chem 272:20275-20282; Yang et al. (1998) J Clin Endocrinol Metab 83(7):2593-6; and Ball et al. (1998) Biol Reprod 59:828-835.)

Despite existing knowledge regarding CTGF and its various biological activities and disease associations, methods of reliably detecting CTGF as distinguished and distinct from fragments of CTGF, and of quantitatively determining the levels of CTGF and of fragments of CTGF in biological samples, have not been available. Current methods of detecting CTGF have, for example, relied on heparin to isolate and concentrate CTGF polypeptides, or involved ELISA assays that did not distinguish between various fragments of CTGF. (See, e.g., Sato et al. (2000) J Rheumatology 27:149-153; Tamanti et al. (1998) Biochem Biophys Res Commun 251:748-752; and Riser et al. (2000) J Am Soc Nephrol 11:25-38.)

Thus, there is a need for assay systems useful in detecting, measuring, and quantifying the levels of CTGF and of various forms of CTGF, such as fragments of CTGF, in biological samples. Specifically, there is a need for methods that selectively identify specific fragments of CTGF as distinguished from CTGF, and that allow for identification and quantitation of specific CTGF fragments implicated in various disease states.

### SUMMARY OF THE INVENTION

Described herein are methods of detecting CTGF and fragments of CTGF, methods of quantitating CTGF and CTGF fragment levels, and methods of diagnosing, determining the prognosis of, and monitoring the efficacy of treatments of various CTGF-associated conditions, diseases, or disorders.

In one aspect, the present disclosure describes methods for detecting CTGF. One method comprises detecting the presence of CTGF N-terminal fragments in a sample, wherein CTGF N-terminal fragments are distinguished from CTGF C-terminal fragments and CTGF. Another method comprises detecting the presence of CTGF C-terminal fragments in a sample, wherein CTGF C-terminal fragments are distinguished from CTGF N-terminal fragments and CTGF. A further method comprises detecting the presence of CTGF in a sample, wherein CTGF is distinguished from CTGF N-terminal and C-terminal fragments.

The disclosure further provides methods for quantitating levels of CTGF. One method comprises quantitating the level of CTGF N-terminal fragments in a sample, wherein CTGF N-terminal fragments are distinguished from CTGF C-terminal fragments and CTGF. Another method comprises quantitating the level of CTGF C-terminal fragments in a sample, wherein CTGF C-terminal fragments are distinguished from CTGF N-terminal fragments and CTGF. A further method comprises quantitating the level of CTGF in a sample, wherein the level of CTGF is distinguished from the levels of CTGF N-terminal and C-terminal fragments.

Some methods comprise contacting a sample with a first reagent which specifically binds to a region on CTGF, isolating the reagent, and quantitating the level of CTGF bound. Additionally, the method may further comprise adding a second reagent selected from a second reagent that specifically binds the first reagent or a second reagent that specifically binds to a region of CTGF different from the region bound by the first reagent, removing unbound second reagent, and quantifying the amount of second reagent bound. In particular methods, the first and second reagents are anti-CTGF antibodies which bind to different regions of CTGF, N-terminal fragments of CTGF, or C-terminal fragments of CTGF. In other specific methods, the first or second reagent is heparin optionally linked to a carrier.

The present invention also contemplates methods for diagnosing or prognosing CTGF-associated disorders, as defined in claim 1. The method comprises quantitating the level of CTGF N-terminal fragments in a sample, and comparing that level to a standard level of CTGF N-terminal fragments, wherein an increased or decreased amount of CTGF N-terminal fragments in the sample is indicative of the presence of a CTGF-associated disorder. For example, an increase in CTGF N-terminal fragments in a sample may be indicative of a fibrotic disorder such as renal fibrosis, liver fibrosis, cardiac fibrosis, scleroderma, and inflammatory joint disease; and a higher level of CTGF N-terminal fragments in a sample compared to a standard may be indicative of a poor prognosis in conditions such as cancer, diabetes, organ transplant, peritoneal dialysis, or myocardial infarction.

Methods for monitoring the progression of a CTGF-associated disorder are also provided, as defined in claim 2. The method comprises comparing the level of CTGF N-terminal fragments in a first sample obtained from a subject at a first point in time to the level of CTGF N-terminal fragments in a second sample obtained from the subject at a second point in time, wherein a difference between the level of CTGF N-terminal fragments in the first sample and the level of CTGF N-terminal fragments in the second sample is indicative of the progression of a CTGF-associated disorder. Similar methods, wherein the levels of CTGF C-terminal fragments or the levels of CTGF in a first and second sample are compared, are also contemplated.

The present disclosure additionally describes methods for monitoring the therapeutic efficacy of treatment of a CTGF-associated disorder. Various methods comprise obtaining a sample from a subject having a CTGF-associated disorder and receiving treatment for that disorder; quantitating the levels of CTGF, or of CTGF N-terminal fragments, or of CTGF C-terminal fragments in the sample; and comparing the level of CTGF, or of CTGF N-terminal fragments, or of CTGF C-terminal fragments to a standard level, wherein a difference between the level determined in the sample and the standard level is indicative of the therapeutic efficacy of treatment of a CTGF-associated disorder. The standard level can be obtained through quantitation of CTGF, of N-terminal fragments of CTGF, or of C-terminal fragments of CTGF in a sample obtained from the same subject at an earlier point in or prior to the beginning of treatment.

In any of the foregoing methods, the sample may be obtained from any source. In one aspect, the sample is obtained from a mammal, and in one particular embodiment the mammal is a human. In one embodiment, the sample may be a body fluid such as urine or plasma. Further, the methods of detection or quantitation of CTGF can be combined with detection and quantitation of additional markers to further confirm diagnosis, prognosis, etc. of a subject's condition. For example, measuring albumin excretion rate in urine in combination with quantitating CTGF N-terminal fragments may provide further confirmation of kidney disease.

The invention further provides kits for use in detecting the presence of or quantitating the level of CTGF, as defined in claim 12. The kits may be used, for example, to diagnose a CTGF-associated disorder. The kit comprises a first antibody specifically reactive with a CTGF N-terminal fragment region from residue 1 to residue 198 of SEQ ID No.2, and a second antibody that is specifically reactive with a CTGF N-terminal fragment region from residue 1 to residue 198 of SEQ ID No.2. The first and second antibodies bind to different regions of N-terminal fragments of CTGF. In other specific embodiments, the first or second reagent is heparin optionally linked to a carrier.

The disclosure further describes methods of screening for compounds that affect the level of CTGF, or of CTGF N-terminal fragments, or of CTGF C-terminal fragments. Certain methods comprise obtaining a sample, quantitating a first level of CTGF, or of CTGF N-terminal fragments, or of CTGF C-terminal fragments in the sample, contacting the sample with a compound, measuring a second level of CTGF, or of CTGF N-terminal fragments, or of CTGF C-terminal fragments; and comparing the first and second levels, wherein a difference between the first level and the second level is indicative of a compound that affects the level of CTGF, or of CTGF N-terminal fragments, or of CTGF C-terminal fragments.

The disclosure further describes methods for identifying a predisposition to a CTGF-associated disorder. Certain methods comprise obtaining a sample, quantitating the level of CTGF N-terminal fragments, CTGF C-terminal fragments, or CTGF in a sample, and comparing the level of N-terminal fragments, C-terminal fragments, or CTGF in the sample with a standard level, wherein an increased or decreased amount of N-terminal fragments of CTGF, C-terminal fragments of CTGF, or CTGF in the sample is indicative of a predisposition to a CTGF-associated disorder.

The present disclosure further describes methods for determining whether a particular disorder has an association with CTGF, i.e., identifying a CTGF-associated disorder. Certain methods comprise obtaining a first sample from a subject having a particular disorder, quantitating the level of CTGF N-terminal fragments, or of CTGF C-terminal fragments, or of CTGF in the sample, and comparing that level to a standard non-disease level, wherein a difference between the level in the first sample and the standard non-disease level is indicative of the presence of a CTGF-associated disorder. The disorder can be one associated with increased levels of CTGF N-terminal fragments, CTGF C-terminal fragments, or CTGF; or one associated with decreased levels of CTGF N-terminal fragments, CTGF C-terminal fragments, or CTGF.

### BRIEF DESCRIPTION OF THE DRAWINGS

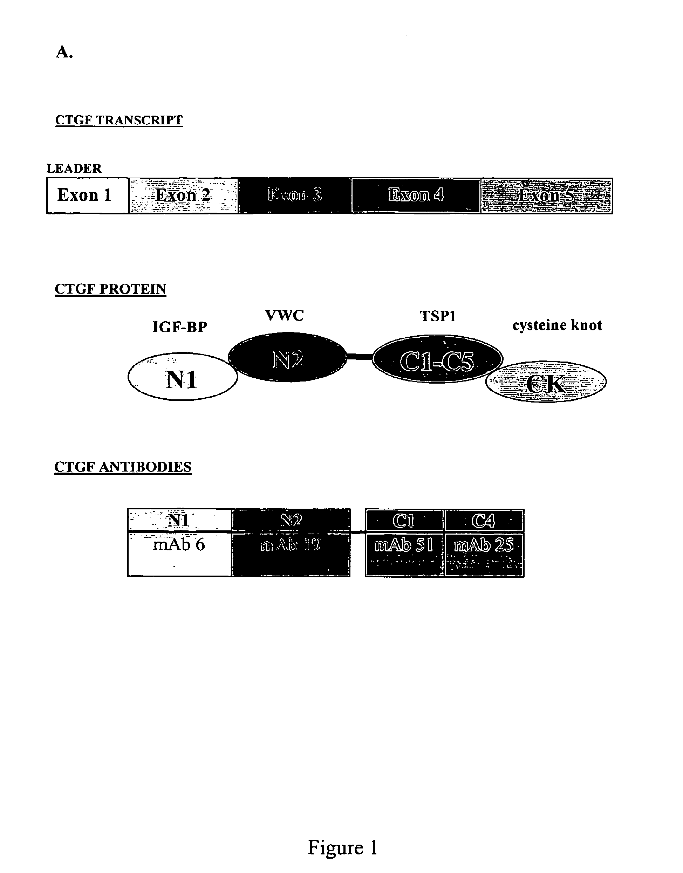

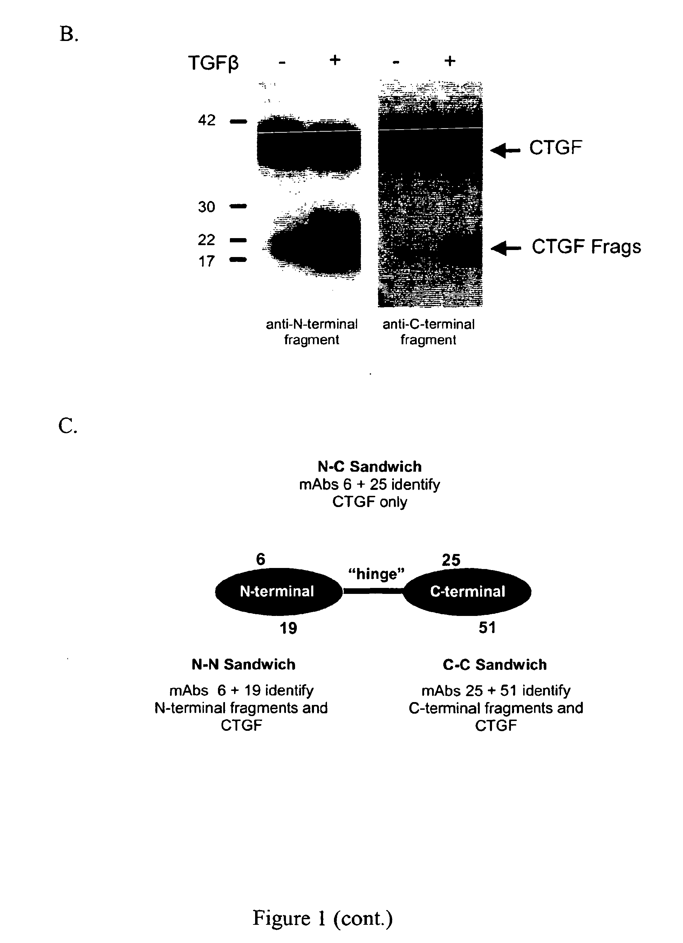

Figures 1A, 1B, and 1C set forth the modular structure of CTGF, a description of particular assays, and exemplary reagents of the present invention. Figure 1A shows the exon structure of the polynucleotide transcript encoding CTGF, the domain structure of the CTGF protein, and exemplary antibody reagents that specifically bind to epitopes on the N-terminal or C-terminal fragment of CTGF. Figure 1B shows a Western blot demonstrating the specificity of reagents that specifically bind to the N-terminal fragment of CTGF or C-terminal fragment of CTGF. Figure 1C shows various dual-reagent "sandwich" assay formats contemplated by the present invention and exemplary reagents that can be used in each assay.

Figure 2 sets forth data showing the specificity of an ELISA assay detecting levels of CTGF (N-C sandwich assay), as distinguished from N-terminal fragments and C-terminal fragments of CTGF.

Figure 3 sets forth data showing the specificity of an ELISA assay detecting levels of CTGF and N-terminal fragments of CTGF (N-N sandwich assay), as distinguished from C-terminal fragments of CTGF.

Figure 4 sets forth data showing the specificity of an ELISA assay detecting CTGF and C-terminal fragments of CTGF (C-C sandwich assay), as distinguished from N-terminal fragments of CTGF.

Figures 5A, 5B, and 5C set forth data showing the levels of CTGF and fragments of CTGF in cell culture supernatants by western blot analysis.

Figure 6 sets forth results showing that N-terminal fragments of CTGF demonstrate greater stability in normal human urine than CTGF.

Figures 7A and 7B set forth data showing the levels of N-terminal fragments of CTGF in dialysate derived from subjects undergoing peritoneal dialysis. Patients were diagnosed with type 1 diabetes, glomerular nephritis, or polycystic fibrosis.

Figure 8 sets forth data showing the levels of CTGF and fragments of CTGF in serum samples derived from renal fibrosis patients compared to that in normal, healthy individuals. The underlying cause of fibrosis was diagnosed as transplant rejection, chemical toxicity, or autoimmune fibrosis.

Figure 9 sets forth data showing the levels of CTGF and fragments of CTGF in serum samples derived from organ transplant patients and from patients with chronic organ transplant rejection.

Figure 10 sets forth data showing the levels of CTGF and fragments of CTGF in serum from patients with myocardial infarction or progressive liver fibrosis compared to that in normal, healthy individuals.

Figure 11 sets forth data showing the level of CTGF and fragments of CTGF in synovial fluid from individuals with inflammatory joint diseases compared to that in normal serum.

Figures 12A and 12B set forth data showing the level of CTGF and fragments of CTGF in vitreous fluid from subjects with various eye diseases.

Figure 13 sets forth data showing the levels of N-terminal fragments of CTGF in serum samples derived from individuals with various cancers.

Figures 14A and 14B set forth data showing the levels ofN-terminal fragments of CTGF in urine from individuals with type 1 diabetes. Figure 12A shows levels of N-terminal fragments of CTGF in patients with type 1 diabetes having no albuminuria, microalbuminuria, or macroalbuminuria. Figure 12B shows a correlation between the level of N-terminal fragments of CTGF in urine and the rate of albumin excretion into urine in patients with type 1 diabetes.

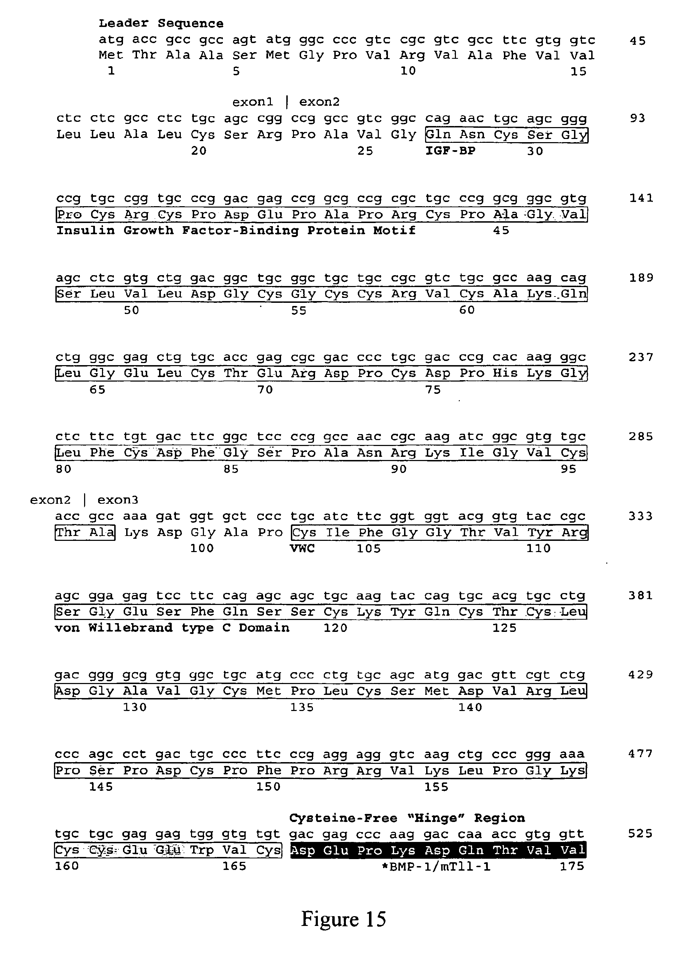

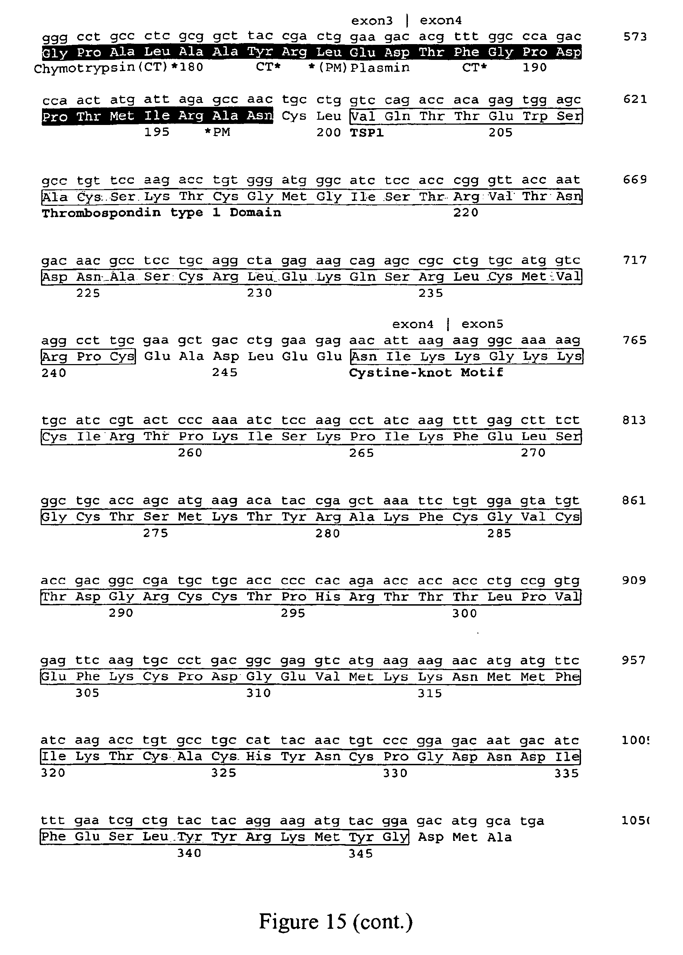

Figure 15 shows a nucleic acid sequence (SEQ ID NO:1) and amino acid sequence (SEQ ID NO:2) of human CTGF. Domains are boxed and shaded, and are identified as follows: IGF-BP (Insulin-like Growth Factor Binding Protein motif), VWC (von Willebrand type C domain), and TSP1 (thrombospondin type 1 domain). Boundaries for neighboring exons are also shown.

Figure 16 shows an alignment between the amino acid sequence of human CTGF (hCTGF; SEQ ID NO:2) and orthologous cow (bCTGF), pig (pCTGF), rat (rCTGF), and mouse (FISP12) CTGF sequences. The alignment was made using the CLUSTALW multiple sequence alignment program (v. 1.74; Thompson et al. (1994) Nucleic Acids Res 22:4673-4680).

### DESCRIPTION OF THE INVENTION

Before the present compositions and methods are described, it is to be understood that the invention is not limited to the particular methodologies, protocols, cell lines, assays, and reagents described, as these may vary. It is also to be understood that the terminology used herein is intended to describe particular embodiments of the present invention.

It must be noted that as used herein and in the appended claims, the singular forms "a," "an," and "the" include plural references unless context clearly dictates otherwise. Thus, for example, a reference to "a fragment" includes a plurality of such fragments, a reference to an "antibody" is a reference to one or more antibodies and to equivalents thereof known to those skilled in the art, and so forth.

Unless defined otherwise, all technical and scientific terms used herein have the same meanings as commonly understood by one of ordinary skill in the art to which this invention belongs. Although any methods and materials similar or equivalent to those described herein can be used in the practice or testing of the present invention, the preferred methods, devices, and materials are now described. All publications cited herein are incorporated herein by reference in their entirety for the purpose of describing and disclosing the methodologies, reagents, and tools reported in the publications which might be used in connection with the invention. Nothing herein is to be construed as an admission that the invention is not entitled to antedate such disclosure by virtue of prior invention.

The practice of the present invention will employ, unless otherwise indicated, conventional methods of chemistry, biochemistry, molecular biology, cell biology, genetics, immunology and pharmacology, within the skill of the art. Such techniques are explained fully in the literature. See, e.g., Gennaro (1990) Remington's Pharmaceutical Sciences, 18th ed., Mack Publishing Co.; Colowick et al., Methods In Enzymology, Academic Press, Inc.; Weir and Blackwell (1986) Handbook of Experimental Immunology, Vols. I-IV, Blackwell Scientific Publications; Maniatis et al. (1989) Molecular Cloning: A Laboratory Manual, 2nd edition, Vols. I-III, Cold Spring Harbor Laboratory Press; Ausubel et al. (1999) Short Protocols in Molecular Biology, 4th edition, John Wiley & Sons; Ream et al. (1998) Molecular Biology Techniques: An Intensive Laboratory Course, Academic Press); Newton and Graham (1997) PCR (Introduction to Biotechniques Series), 2nd ed., Springer Verlag).

### DEFINITIONS

"Connective tissue growth factor" or "CTGF" refers to the amino acid sequences of substantially purified CTGF derived from any species, particularly a mammalian species, including rat, rabbit, bovine, ovine, porcine, murine, equine, and particularly human species, and from any source, whether natural, synthetic, semi-synthetic, or recombinant.

In one aspect, "connective tissue growth factor" or "CTGF" refers to a polypeptide sequence comprising at least a portion of the N-terminal fragment of CTGF and at least a portion of the C-terminal fragment of CTGF.

The terms "N-terminal fragment" and "N-fragment" used in reference to CTGF mean any polypeptide comprising sequences derived from the amino-terminal portion of a CTGF polypeptide, or to any variants, or fragments thereof. CTGF N-terminal fragments can include all, none, or portions of CTGF from the initial methionine residue through the cysteine-free "hinge" region. Further, CTGF N-terminal fragments can include all, none, or portions of the insulin growth factor-binding protein motif and/or the von Willebrand type C domain. N-terminal fragments of CTGF can also include all, none, or portions of the cysteine-free region. Further, N-terminal fragments of CTGF can be any fifteen or more contiguous amino acids contained within any preceding CTGF N-terminal fragment defined above.

In one aspect, "N-terminal fragment" or "N-fragment" of CTGF refers to polypeptide sequences derived from the amino-terminal portion of human CTGF. Such fragments can encompass the entire region from amino acid residue 1 to about amino acid residue 198 of SEQ ID NO:2, or from about amino acid 23 to about amino acid 198 of SEQ ID NO:2. The boundary of the N-terminal fragment within the hinge region may be optionally defined by one of several protease cleavage sites defined in SEQ ID NO:2, such as chymotrypsin cleavage sites between residues 179 and 180, between residues 182 and 183, and between residues 188 and 189 of SEQ ID NO:2; plasmin cleavage sites between residues 183 and 184, and between residues 196 and 197 of SEQ ID NO:2; and a bone morphogenetic protein-1 cleavage site between residues 169 and 170 of SEQ ID NO:2. Additionally, N-terminal fragments of human CTGF can include all, none, or portions of the region from amino acid 27 to amino acid 97 of SEQ ID NO:2, amino acid 103 to amino acid 166 of SEQ ID NO:2, or amino acid 167 to amino acid 198 of SEQ ID NO:2. Further, N-terminal fragments of human CTGF can be any fifteen or more contiguous amino acids contained within any preceding CTGF N-terminal fragment defined above.

In specific embodiments, the CTGF N-terminal fragments of the present invention comprise sequences selected from the following regions of human CTGF (SEQ ID NO:2) and orthologous fragments thereof derived from a different species, particularly a mammalian species including rat, rabbit, bovine, ovine, porcine, murine, and equine: amino acid residue 23 to amino acid residue 96 (encoded by exon 2); amino acid residue 27 to amino acid residue 97 (IGF-BP motif); amino acid residue 97 to amino acid residue 180 (encoded by exon 3); amino acid residue 103 to amino acid residue 166 (VWC domain); amino acid residue 167 to amino acid residue 198 (cysteine-free hinge) not encompassed by a corresponding C-terminal fragment of CTGF; amino acid residue 23 to amino acid residue 180 (encoded by exons 2 and 3); amino acid residue 27 to amino acid residue 166 (IGF-BP and VWC domains); and amino acid residue 23 to amino acid residue 198.

The terms "C-terminal fragment" and "C-fragment" used in reference to CTGF mean any polypeptide comprising sequences derived from the carboxy-terminal portion of a CTGF polypeptide, or to any variants, or fragments thereof. C-terminal fragments can include all, none, or portions of CTGF from the cysteine-free hinge region to the end of the protein. Further, CTGF C-terminal fragments can include all, none, or portions of the Thrombospondin Type 1 domain and/or the Cysteine-Knot motif. Further, C-terminal fragments of CTGF can be any fifteen or more contiguous amino acids contained within any preceding CTGF C-terminal fragment defined above.

In one aspect, "C-terminal fragment" or "C-fragment" of CTGF refers to polypeptide sequences derived from the carboxy-terminal portion of human CTGF. Such fragments can encompass the entire region from amino acid residue 181 to about amino acid residue 349 of SEQ ID NO:2. The boundary of the C-terminal fragment within the hinge region may be optionally defined by one of several protease cleavage sites defined in SEQ ID NO:2, such as chymotrypsin, plasmin, and bone morphogenetic protein-1 cleavage sites defined above. Additionally, C-terminal fragments of human CTGF can include all, none, or portions of the region from amino acid 201 to amino acid 242 of SEQ ID NO:2, amino acid 247 to amino acid 349 of SEQ ID NO:2, amino acid 248 to amino acid 349 of SEQ ID NO:2, or amino acid 249 to amino acid 346 of SEQ ID NO:2. Further, C-terminal fragments of human CTGF can be any fifteen or more contiguous amino acids contained within any preceding CTGF C-terminal fragment defined above.

In specific embodiments, the CTGF C-terminal fragments of the present invention comprise sequences selected from the following regions of human CTGF (SEQ ID NO:2) and orthologous fragments thereof derived from a different species, particularly a mammalian species including rat, rabbit, bovine, ovine, porcine, murine, and equine: amino acid residue 181 to amino acid residue 251 (encoded by exon 4); amino acid residue 201 to amino acid residue 242 (thrombospondin type 1 motif); amino acid residue 252 to amino acid residue 349 (encoded by exon 5); amino acid residue 249 to amino acid residue 346 (cysteine knot domain); a portion of amino acid residue 167 to amino acid residue 198 (cysteine-free hinge) not encompassed by a corresponding N-terminal fragment of CTGF; amino acid residue 181 to amino acid residue 348 (encoded by exons 2 and 3); amino acid residue 201 to amino acid residue 346 (TP1 and CK domains); amino acid residue 247 to amino acid residue 348; and amino acid residue 248 to amino acid residue 348.

The terms "cysteine-free region" or "hinge region" of CTGF refer to any polypeptide derived from about amino acid residue 167 to about amino acid residue 198 of human CTGF (SEQ ID NO:2) and orthologous fragments thereof derived from a different species, particularly a mammalian species including rat, rabbit, bovine, ovine, porcine, murine, and equine.

"Amino acid sequence" or "polypeptide" or "polypeptides," as these terms are used herein, refer to oligopeptide, peptide, polypeptide, or protein sequences, and fragments thereof, and to naturally occurring or synthetic molecules. Polypeptide or amino acid fragments are any portion of a polypeptide which retains at least one structural and/or functional characteristic of the polypeptide. CTGF fragments include any portion of a CTGF polypeptide sequence which retains at least one structural or functional characteristic of CTGF. Where "amino acid sequence" refers to the polypeptide sequence of a naturally occurring protein molecule, "amino acid sequence" and like terms are not meant to limit the amino acid sequence to the complete native sequence associated with the protein molecule in question.

The terms "nucleic acid" or "polynucleotide" or "polynucleotides" refer to oligonucleotides, nucleotide sequences, or polynucleotides, or any fragments thereof, and to DNA or RNA of natural or synthetic origin which may be single- or double-stranded and may represent the sense or antisense strand, to peptide nucleic acid (PNA), or to any DNA-like or RNA-like material, natural or synthetic in origin. Polynucleotide fragments are any portion of a polynucleotide sequence that retains at least one structural or functional characteristic of the polynucleotide. Polynucleotide fragments can be of variable length, for example, greater than 60 nucleotides in length, at least 100 nucleotides in length, at least 1000 nucleotides in length, or at least 10,000 nucleotides in length.

"Altered" polynucleotides include those with deletions, insertions, or substitutions of different nucleotides resulting in a polynucleotide that encodes the same or a functionally equivalent polypeptide. Included within this definition are sequences displaying polymorphisms that may or may not be readily detectable using particular oligonucleotide probes or through deletion of improper or unexpected hybridization to alleles, with a locus other than the normal chromosomal locus for the subject polynucleotide sequence.

"Altered" polypeptides may contain deletions, insertions, or substitutions of amino acid residues which produce a silent change and result in a functionally equivalent polypeptide. Deliberate amino acid substitutions may be made on the basis of similarity in polarity, charge, solubility, hydrophobicity, hydrophilicity, and/or the amphipathic nature of the residues as long as the biological or immunological activity of the encoded polypeptide is retained. For example, negatively charged amino acids may include aspartic acid and glutamic acid; positively charged amino acids may include lysine and arginine; and amino acids with uncharged polar head groups having similar hydrophilicity values may include leucine, isoleucine, and valine, glycine and alanine, asparagine and glutamine, serine and threonine, and phenylalanine and tyrosine.

A polypeptide or amino acid "variant" is an amino acid sequence that is altered by one or more amino acids from a particular amino acid sequence. A polypeptide variant may have conservative changes, wherein a substituted amino acid has similar structural or chemical properties to the amino acid replaced, *e.g.,* replacement of leucine with isoleucine. A variant may also have non-conservative changes, in which the substituted amino acid has physical properties different from those of the replaced amino acid, e.g., replacement of a glycine with a tryptophan. Analogous minor variations may also include amino acid deletions or insertions, or both. Preferably, amino acid variants retain certain structural or functional characteristics of a particular polypeptide. Guidance in determining which amino acid residues may be substituted, inserted, or deleted may be found, for example, using computer programs well known in the art, such as LASERGENE software (DNASTAR Inc., Madison, WI).

A polynucleotide variant is a variant of a particular polynucleotide sequence that preferably has at least about 80%, more preferably at least about 90%, and most preferably at least about 95% polynucleotide sequence similarity to the particular polynucleotide sequence. It will be appreciated by those skilled in the art that as a result of the degeneracy of the genetic code, a multitude of variant polynucleotide sequences encoding a particular protein, some bearing minimal homology to the polynucleotide sequences of any known and naturally occurring gene, may be produced. Thus, the invention contemplates each and every possible variation of polynucleotide sequence that could be made by selecting combinations based on possible codon choices. These combinations are made in accordance with the standard codon triplet genetic code, and all such variations are to be considered as being specifically disclosed.

A "deletion" is a change in an amino acid or nucleotide sequence that results in the absence of one or more amino acid residues or nucleotides.

The terms "insertion" or "addition" refer to a change in a polypeptide or polynucleotide sequence resulting in the addition of one or more amino acid residues or nucleotides, respectively, as compared to the naturally occurring molecule.

The term "functional equivalent" as it is used herein refers to a polypeptide or polynucleotide that possesses at least one functional and/or structural characteristic of a particular polypeptide or polynucleotide. A functional equivalent may contain modifications that enable the performance of a specific function. The term "functional equivalent" is intended to include fragments, mutants, hybrids, variants, analogs, or chemical derivatives of a molecule.

The term "microarray" refers to any arrangement of molecules, e.g. nucleic acids, amino acids, antibodies, etc., on a substrate. The substrate can be any suitable support, e.g., beads, glass, paper, nitrocellulose, nylon, or any appropriate membrane, etc. A substrate can be any rigid or semi-rigid support including, but not limited to, membranes, filters, wafers, chips, slides, fibers, beads, including magnetic or nonmagnetic beads, gels, tubing, plates, polymers, microparticles, capillaries, etc. The substrate can provide a surface for coating and/or can have a variety of surface forms, such as wells, pins, trenches, channels, and pores, to which the nucleic acids, amino acids, etc., may be bound.

"Antigenicity" relates to the ability of a substance to, when introduced into the body, stimulate the immune response and the production of an antibody. An agent displaying the property of antigenicity is referred to as being antigenic. Antigenic agents can include, but are not limited to, a variety of macromolecules such as, for example, proteins, lipoproteins, polysaccharides, nucleic acids, bacteria and bacterial components, and viruses and viral components. Antigenic fragments refer to fragments of CTGF polypeptide, preferably, fragments of about five to fifteen amino acids in length, that retain at least one biological or immunological aspect of CTGF polypeptide activity.

"Immunogenicity" relates to the ability to evoke an immune response within an organism. An agent displaying the property of immunogenicity is referred to as being immunogenic. Agents can include, but are not limited to, a variety of macromolecules such as, for example, proteins, lipoproteins, polysaccharides, nucleic acids, bacteria and bacterial components, and viruses and viral components. Immunogenic agents often have a fairly high molecular weight (usually greater than 10 kDa). Immunogenic fragments refer to fragments of CTGF polypeptide, preferably, fragments of about five to fifteen amino acids in length, that retain at least one biological or immunological aspect of CTGF polypeptide activity.

The term "antibody" refers immunoglobulins or antibodies obtained from any source including a cell line, or an animal such as mouse, rat, rabbit, chicken, turkey, goat, horse, human, etc. Antibodies may also be obtained from genetically modified cells, or transgenic plants or animals engineered to make antibodies that are not endogenous to the host. An antibody may be of any isotype including IgA, IgD, IgE, IgG-1, IgG-2, IgG-3, IgG-4, or IgM. Further, the term "antibody" includes intact molecules and fragments thereof, such as Fab, F(ab')₂, and Fv fragments, which are capable of binding the epitopic determinant, and includes polyclonal or monoclonal antibodies. Antibody can also refer to chimeric antibodies, e.g., bivalent and trivalent antibodies, that bind to one or more unique antigen(s).

Antibodies that bind CTGF or fragments of CTGF can be prepared using intact polypeptides or using fragments containing small peptides of interest as the immunizing antigen. The polypeptide or oligopeptide used to immunize an animal can be derived from the translation of RNA, or synthesized chemically, and can be conjugated to a carrier protein if desired. Commonly used carriers chemically coupled to peptides include, for example, bovine serum albumin, thyroglobulin, and keyhole limpet hemocyanin (KLH).

The terms "disorders" and "diseases" are used inclusively and refer to any condition deviating from normal.

The phrase "CTGF-associated disorders" as used herein refers to conditions and diseases associated with abnormal or inappropriate expression or activity of CTGF. Abnormal expression of CTGF has been associated with cell proliferative disorders, such as those caused by endothelial cell proliferation or migration, tumor-like growths, general tissue scarring, and various diseases characterized by inappropriate deposition of extracellular matrix.

CTGF-associated disorders include, but are not limited to, disorders involving angiogenesis and other proliferative processes which play central roles in conditions such as atherosclerosis, glaucoma, etc.; and cancer, including acute lymphoblastic leukemia, dermatofibromas, breast cancer, breast carcinoma desmoplasia, angiolipoma, angioleiomyoma, desmoplastic cancers, and prostate, ovarian, colorectal, pancreas, gastrointestinal, and liver cancer, and other tumor growth and metastases.

Further, CTGF-associated disorders include, but are not limited to, excessive scarring resulting from localized or systemic fibrosis, including chronic or acute fibrosis of organs such as the kidney, lungs, liver, eyes, heart, skin, etc. Such CTGF disorders include various fibrotic disorders, such as, for example, cardiac fibrosis, including cardiac reactive fibrosis or cardiac remodeling following myocardial infarction or congestive heart failure; pulmonary disorders, including interstitial pulmonary fibrosis, etc.; fibrosis associated with dialysis including peritoneal dialysis, e.g., continuous ambulatory peritoneal dialysis (CAPD); peridural fibrosis; kidney fibrosis; pulmonary fibrosis; interstitial fibrosis; skin fibrosis; and fibrosis resulting from acute or repetitive traumas, including surgery, chemotherapy, radiation treatment, allograft rejection, chronic and acute transplant rejection (e.g., kidney, liver, or other organ); bronchiolitis obliterans, e.g., following lung transplant; and inflammation and infection, e.g., due to disease or injury.

Additionally, CTGF-associated disorders include, but are not limited to, sclerotic conditions, including systemic sclerosis, scleroderma, keloids, hypertrophic scarring, and other dermatological diseases and conditions; atherosclerosis, such as conditions involving atherosclerotic plaques and atherosclerosis associated with diabetes, including atherosclerosis associated with peritoneal dialysis; arteriosclerosis; arthritis, including rheumatoid arthritis, osteoarthritis, and other joint inflammatory conditions, etc.; interstitial diseases, including interstitial fibrosis; Crohn's disease; inflammatory bowel disease; retinopathies, including, for example, proliferative vitreoretinopathy, non-proliferative diabetic retinopathy, proliferative diabetic retinopathy, and macular degeneration (including age-related and juvenile (Stargardt's) disease, and pigment epithelial detachment); nephropathies, including diabetic nephropathy, IgA-associated nephropathy, nephropathy due to toxicity, etc.; and conditions associated with chemical toxicity tubule destruction.

The "proliferative" processes and disorders referred to herein include pathological states characterized by the continual multiplication of cells resulting in an overgrowth of a cell population within a tissue. The cell populations are not necessarily transformed, tumorigenic or malignant cells, but can include normal cells as well. For example, CTGF may be involved pathologically by inducing a proliferative lesion in the intimal layer of an arterial wall, resulting in atherosclerosis, or by stimulating neovascularization.

"Cancer" refers to any autonomous growth of tissue, including uncontrolled, abnormal growth of cells, or to any malignant tumor of potentially unlimited growth that expands locally by invasion and systemically by metastasis. Cancer also refers to any abnormal state marked by a cancer.

The term "fibrosis" refers to abnormal processing of fibrous tissue, or fibroid or fibrous degeneration. Fibrosis can result from various injuries or diseases, and can often result from chronic transplant rejection relating to the transplantation of various organs. Fibrosis typically involves the abnormal production, accumulation, or deposition of extracellular matrix components, including overproduction and increased deposition of, for example, collagen and fibronectin. "Fibrosis" is used herein in its broadest sense referring to any excess production or deposition of extracellular matrix proteins. There are numerous examples of fibrosis, including the formation of scar tissue following a heart attack, which impairs the ability of the heart to pump. Diabetes frequently causes damage/scarring in the kidneys which leads to a progressive loss of kidney function. Even after surgery, scar tissue can form between internal organs causing contracture, pain, and in some cases, infertility. Major organs such as the heart, kidney, liver, eye, and skin are prone to chronic scarring, commonly associated with other diseases. Hypertrophic scars (non-malignant tissue bulk) are a common form of fibrosis caused by burns and other trauma. In addition, there are a number of other fibroproliferative disorders, including scleroderma, keloids, and atherosclerosis, which are associated respectively with general tissue scarring, tumor-like growths in the skin, or sustained scarring of blood vessels which impairs blood carrying ability.

The term "sample" is used herein in its broadest sense. Samples may be derived from any source, for example, from bodily fluids, secretions, tissues, cells, or cells in culture including, but not limited to, saliva, blood, urine, serum, plasma, vitreous, synovial fluid, cerebral spinal fluid, amniotic fluid, and organ tissue (e.g., biopsied tissue); from chromosomes, organelles, or other membranes isolated from a cell; from genomic DNA, cDNA, RNA, mRNA, etc.; and from cleared cells or tissues, or blots or imprints from such cells or tissues. Samples may be derived from any source, such as, for example, a human subject, or a non-human mammalian subject, etc. Also contemplated are samples derived from any animal model of disease. A sample can be in solution or can be, for example, fixed or bound to a substrate. A sample can refer to any material suitable for testing for the presence of CTGF or of fragments of CTGF or suitable for screening for molecules that bind to CTGF or to fragments thereof. Methods for obtaining such samples are within the level of skill in the art.

The term "hybridization" refers to the process by which a nucleic acid sequence binds to a complementary sequence through base pairing. Hybridization conditions can be defined by, for example, the concentrations of salt or formamide in the prehybridization and hybridization solutions, or by the hybridization temperature, and are well known in the art. Hybridization can occur under conditions of various stringency.

In particular, reducing the concentration of salt, increasing the concentration of formamide, or raising the hybridization temperature can increase stringency. For example, for purposes of the present invention, hybridization under high stringency conditions might occur in about 50% formamide at about 37°C to 42°C, and under reduced stringency conditions in about 35% to 25% formamide at about 30°C to 35°C. In particular, hybridization generally occurs in conditions of highest stringency at 42°C in 50% formamide, 5X SSPE, 0.3% SDS, and 200 µg/ml sheared and denatured salmon sperm DNA.

The temperature range corresponding to a particular level of stringency can be further narrowed by methods known in the art, for example, by calculating the purine to pyrimidine ratio of the nucleic acid of interest and adjusting the temperature accordingly. To remove nonspecific signals, blots can be sequentially washed, for example, at room temperature or up to and including 60°C, under increasingly stringent conditions of up to 0.1X SSC and 0.5% SDS. Variations on the above ranges and conditions are well known in the art.

### INVENTION

Presented herein is the discovery that specific fragments of CTGF can be detected and, further, can be quantitated, independently of CTGF or of other CTGF fragments. The present invention provides methods of reliably detecting and quantitating the levels of N-terminal fragments of CTGF in biological samples. Furthermore, diagnosis, prognosis, and determination of the progression of various diseases and conditions, for example, diabetes; fibrosis including liver, renal, and pulmonary fibrosis; myocardial infarction; inflammatory joint disease; cancer; systemic sclerosis; angiogenesis; arteriosclerosis, atherosclerosis; transplant rejection; various eye diseases including diabetic retinopathy; limited and diffuse scleroderma; renal hypertension; and conditions associated with peritoneal dialysis, etc., can also be accomplished using the compositions and methods described herein.

### Connective Tissue Growth Factor

Connective tissue growth factor (CTGF) has been reported and described previously. (See, *e.g.,* U.S. Patent No. 5,408,040; Bradham et al. (1991) J Cell Biology 114:1285-1294.) CTGF is a monomeric polypeptide with a molecular weight of approximately 36 to 38 kDa. CTGF is a member of a recently described family of cysteine-rich secreted proteins called the CCN family of growth factors (CTGF, Cyr-61, nov), characterized by the presence of highly conserved cysteine residues within distinct modular domains. These domains, each of which is encoded by a separate exon, exhibit homology to conserved regions found in a variety of extracellular matrix proteins. Specifically, within CTGF, these modules have structural similarity to the N-terminal cysteine-rich regions of insulin-like growth factor binding proteins (domain 1 of CTGF, encoded by exon 2); the Von Willebrand Factor type C domain, implicated in oligomerization (domain 2 of CTGF, encoded by exon 3); a thrombospondin type I motif, which may contain a cell attachment region and is believed to be involved in binding to extracellular matrix and sulfated glycoconjugates (domain 3 of CTGF, encoded by exon 4); and a C-terminal cysteine knot motif similar to that found in nerve growth factor, transforming growth factor-□ (TGF□), and platelet derived growth factor (PDGF), which may be involved in receptor binding (domain 4 of CTGF, encoded by exon 5). (Bork (1993) FEBS Lett 327:125-130.)

Various fragments of CTGF have been shown to exhibit biological activities. (See, e.g., U.S. Patent No. 5,876,730; Brigstock et al. (1997) J Biol Chem 272:20275-20282; Ball et al. (1998) Biol Reprod 59:828-835; Steffen et al. (1998) Growth Factors 15:199-213; International Application No. WO 00/35939; and International Application No. WO 00/35936.) Fragments of CTGF have also been detected in human biological fluids, such as those derived from pregnancy serum, amniotic fluids, and peritoneal fluids, as well as in the conditioned media of cultured bovine endothelial cells. (Yang et al. (1998) J Clin Endocrinol Metab 83:2593-2596); Boes et al. (1999) Endocrinology 140:1575-1580.)

CTGF acts to promote fibroblast and other connective tissue cell proliferation, chemotaxis, migration, adhesion, and extracellular matrix formation. Evidence suggests aberrant expression or overproduction of CTGF plays a major role in pathways that lead to fibrotic disorders, including fibrosis of major organs, fibroproliferative diseases, and scarring. CTGF produces effects on a variety of cell types, including, for example, connective tissue cells (e.g., fibroblasts, myofibroblasts, etc.), vascular endothelial cells, epithelial cells, neuronal cells, vascular smooth muscle cells, and more specialized connective tissue cells, such as cells of bone, cartilage, and other supportive skeletal tissues. (See, e.g., Moussad and Brigstock (2000) Mol Genet Metab 71:276-292.)

The present disclosure relates to the discovery that levels of certain fragments of CTGF are elevated in certain conditions. The present invention demonstrates that in certain conditions N-terminal fragments of CTGF are more readily detected, e.g., in biological fluids, etc., than CTGF or C-terminal fragments of CTGF. Although the invention is not limited to any particular mechanism by which fragments of CTGF accumulate, the N-terminal fragments may be more stable because they are less prone to proteolytic degradation, less prone to clearance or elimination, and/or less prone to non-specific binding or adsorption. Accordingly, detection of N-terminal fragments of CTGF can provide finer specificity and reproducibility for elucidating the mechanisms and sites of action of this polypeptide. In addition, the present disclosure establishes that aberrant levels of CTGF, particularly N-terminal fragments of CTGF, can be associated with both the presence of disease and the severity of disease. Thus, the present invention provides novel and superior methods for diagnosis, prognosis, and therapeutic monitoring of certain diseases associated with CTGF and CTGF fragment expression.

Accordingly, in one aspect, the present invention relates to use of N-terminal fragments of a CTGF polypeptide for detection (qualitative or quantitative) of CTGF and for prognosis, diagnosis, and therapeutic monitoring of CTGF-associated disorders. N-terminal fragments of CTGF can be detected, identified, and quantified in any number of ways as provided by the present invention.

The present invention contemplates that, in some embodiments, the C-terminal and N-terminal fragments of CTGF are variants of specific C-terminal and N-terminal fragments of CTGF, which may be constructed by mutating the polynucleotide sequences encoding the fragment of interest to give an amino acid sequence that does not occur in nature. Amino acid alterations can be made at sites that differ in subunits from different species (variable positions) or in highly conserved regions (constant regions). Sites at such locations will typically be modified in series, e.g., by substituting first with conservative choices (e.g., hydrophobic amino acid to a different hydrophobic amino acid) and then with more distant choices (e.g., hydrophobic amino acid to a charged amino acid), and then deletions or insertions may be made at the target site.

The polypeptides described herein, for example, those useful in generating CTGF N-terminal or CTGF C-terminal specific antibodies, can be isolated or produced using any of the numerous methods known in the art to synthesize the desired N-terminal or C-terminal fragments of CTGF amino acid sequence at least in part. For example, peptides can be synthesized by solid phase techniques, cleaved from the resin, and purified by preparative high performance liquid chromatography. The composition of the synthetic peptides may be confirmed, for example, by amino acid analysis, mass spectrometry, or sequencing.

Polymerase chain reaction (PCR) may also be used to create amino acid sequence variants of CTGF and fragments thereof. When small amounts of template DNA are used as starting material, a primer or primers that differ slightly in sequence from the corresponding region in the template DNA can generate the desired amino acid variant. PCR amplification results in a population of product DNA fragments that differ from the polynucleotide template encoding the CTGF or fragment thereof at the position specified by the primer. The product DNA fragments replace the corresponding region in the plasmid and this gives the desired amino acid variant. Further techniques for generating amino acid variants include the cassette mutagenesis technique described in Wells et al. (1985, Gene 34:315) and other mutagenesis techniques well known in the art, such as, for example, the techniques described generally in Maniatis, T. et al., *supra;* and Ausubel, F.M. et al., *supra.*

In an alternate embodiment of the invention, the coding sequence of the CTGF polypeptide of the invention can be synthesized in whole or in part, using chemical methods well known in the art. (See, for example, Caruthers et al. (1980) Nucleic Acids Symp Ser 7:215-233; Crea and Horn (1980) Nucleic Acids Res 9:2331; Matteucci and Caruthers (1980) Tetrahedron Letters 21:719; and, Chow and Kempe (1981) Nucleic Acids Res 9:2807-2817.)

### Detection of CTGF, N-terminal Fragments of CTGF, and C-terminal Fragments of CTGF

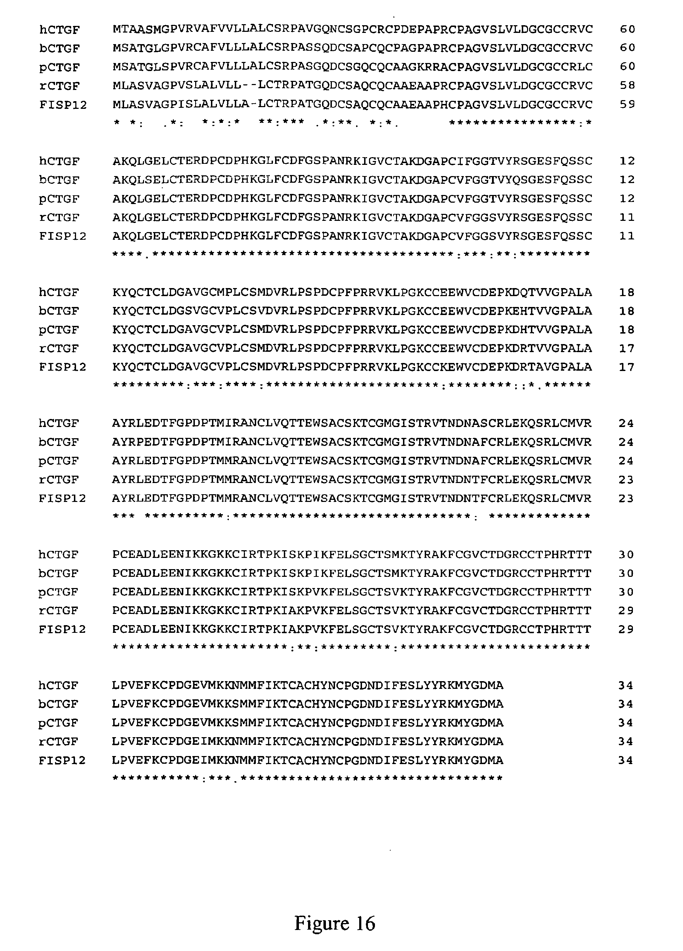

The present disclosure describes methods for detecting and/or quantitating levels of CTGF polypeptides *(e.g.,* CTGF, N-terminal fragments of CTGF, C-terminal fragments of CTGF) in a sample. The methods described are not limited to the detection and quantitation of human CTGF polypeptides, but can be used for the detection of CTGF from other species, such as, for example, detecting and quantitating non-human mammalian CTGF. (See, Figure 16.) For instance, detection and quantitation CTGF, including endogenous CTGF and exogenous CTGF from the same or a different species, may be useful in animal models of CTGF-associated disorders and other various diseases. A method for detecting CTGF in a biological sample is described, the method involving obtaining a sample and detecting in the sample CTGF, N-terminal fragments of CTGF, and C-terminal fragments of CTGF. In another aspect, a method for quantitating the levels of CTGF in a biological sample is described, the method comprising obtaining a sample and quantitating in the sample the levels of CTGF, N-terminal fragments of CTGF, and C-terminal fragments of CTGF. (See, e.g., Figure 1C.)

In one aspect, the assays are based on the detection of N-terminal fragments of CTGF using antibodies produced against these polypeptides. (See, e.g., Figure 1A.) The assays may also include nucleic acid-based assays (typically based upon hybridization), or any other of the various assaying techniques known in the art. In a preferred embodiment, the present methods are characterized by the ability of the polypeptides (e.g., N-terminal fragments of CTGF,) of the present invention to be bound by antibodies specific for the polypeptides, and the ability of antibodies produced against the proteins of the present invention to bind to CTGF polypeptides. Other methods of detecting N-terminal fragments of CTGF standard in the art are contemplated herein. For example, any agent that binds to or interacts with fragments of CTGF could be used, such as heparin, a receptor to CTGF or to fragments of CTGF, or any other CTGF binding agent.

According to the present disclosure, various immunoassay techniques can be used to detect and quantitate the levels of CTGF and of CTGF fragments in a sample. In one embodiment, an ELISA assay is used to identify and distinguish the levels of CTGF and of different CTGF fragments and to compare the amount of CTGF to that of different CTGF fragments within a particular sample. In certain embodiments, the ELISA can be used to detect, quantitate, and compare levels of CTGF and of CTGF fragments found in conditioned media from cultured cells, as well as in blood, serum, plasma, peritoneal effluent, urine, vitreous, synovial fluid, cerebral spinal fluid, saliva, and other biological samples in which detection and quantitation of CTGF and of fragments of CTGF would be desired.

Improved and more sensitive detection and quantitation of CTGF is achieved by detecting N-terminal fragments of CTGF as compared to detecting C-terminal fragments of CTGF or CTGF. The use of N-terminal fragments of CTGF allows for detection of disease severity that permits more accurate diagnosis, prognosis, and monitoring of therapeutic efficacies of CTGF-associated disorders.

The methods provided by the present disclosure are also useful for the detection and quantitation of any of the members of the CCN family of polypeptides (such as, for example, CTGF, CYR61, Nov, Elm1/WISP-1, HICP/rCOP-1, CTGF-3/WISP-2, and WISP-3) and fragments thereof, and in the detection, diagnosis, prognosis, and monitoring of therapeutic efficacy of various diseases and disorders associated with CCN family members. (See, *e.g*., Brigstock (1999) Endocrine Reviews 20:189-206; and Perbal (2001) Mol. Pathol. 54:57-79.)

### Antibodies

In preferred embodiments of the present invention, methods for detection, measurement, diagnosis, prognosis, monitoring, and evaluation of diseases and disorders, and therapeutic treatments thereof, associated with increased or decreased expression or activity of CTGF or fragments thereof involve the use of antibodies which specifically react with a CTGF epitope or epitopes.

Polypeptides used to generate suitable antibodies can be generated in a variety of ways, for example, by generation of synthetic peptides, by enzymatic cleavage of CTGF (e.g., by chymotrypsin treatment), by chemical cleavage of CTGF, by recombinant expression of CTGF or fragments thereof, or by isolation from a human or other mammalian source. Thus, cells that express CTGF (such as MG-63 cells), host cells transfected with CTGF coding sequences, purified CTGF, C-terminal fragments of CTGF, N-terminal fragments of CTGF, or epitopes therein may be used as immunogens to elicit an immune response in animal hosts for the generation of antibodies specific for the desired region of CTGF. For recombinantly produced polypeptides, suitable expression vectors and systems are known to those of skill in the art and include eukaryotic and prokaryotic expression systems, as described, for example, in Maniatis et al., *supra,* and Ausubel et al., *supra.*

Target polypeptides, such as, for example, N-terminal fragments of CTGF or C-terminal fragments of CTGF, can be evaluated to determine regions of high immunogenicity. Methods of analysis and epitope selection are well-known in the art. (See, *e.g.*, Ausubel et al., *supra)* Analysis and selection can also be accomplished, for example, by various software packages, such as LASERGENE NAVIGATOR software. (DNASTAR; Madison, WI.) The peptides or fragments used to induce antibody production should be antigenic, but need not necessarily be biologically active. Preferably, an antigenic fragment or peptide is at least five amino acids in length, more preferably, at least ten amino acids in length, and most preferably, at least fifteen amino acids in length. It is preferable that the antibody-inducing fragment or peptide is identical to at least a portion of the amino acid sequence of the target polypeptide, e.g., N-terminal regions or domains of CTGF or C-terminal regions or domains of CTGF. A peptide or fragment that mimics at least a portion of the sequence of the naturally occurring target polypeptide can also be fused with another protein, *e.g.,* keyhole limpet hemocyanin (KLH), and antibodies can be produced against the chimeric molecule.

Methods for the production of antibodies are well known in the art. For example, various hosts, including goats, rabbits, rats, mice, chickens, turkeys, humans, and others, may be immunized by injection with the target polypeptide or any immunogenic fragment or peptide thereof. Depending on the host species, various adjuvants may be used to increase immunological response. Such adjuvants include, but are not limited to, Freund's adjuvant, mineral gels such as aluminum hydroxide, and surface active substances such as lysolecithin, pluronic polyols, polyanions, peptides, oil emulsions, and KLH. Among adjuvants used in humans, aluminum hydrogels, BCG (bacilli Calmette-Guerin) and *Corynebacterium parvum* are especially preferable.

Monoclonal antibodies may be prepared using any technique which provides for the production of antibody molecules by continuous cell lines in culture. Techniques for *in vivo* and *in vitro* production of either monoclonal or polyclonal antibodies are well known in the art. (See, *e.g.,* Pound (1998) Immunochemical Protocols, Humana Press, Totowa NJ; Harlow and Lane (1988) Antibodies: A Laboratory Manual, Cold Spring Harbor Laboratory, New York; Goding (1986) Monoclonal Antibodies: Principles and Practice, 2nd Edition, Academic Press; Schook (1987) Monoclonal Antibody Production Techniques and Applications, Marcel Dekker, Inc.) The production of chimeric antibodies is also well known in the art, as is the production of single-chain antibodies. (See, *e.g*., Morrison et al. (1984) Proc Natl Acad Sci 81:6851-6855; Neuberger et al. (1984) Nature 312:604-608; Takeda et al. (1985) Nature 314:452-454.) Antibodies with related specificity, but of distinct idiotypic composition, may be generated, for example, by chain shuffling from random combinatorial immunoglobin libraries. (See, e.g., Burton (1991) Proc Natl Acad Sci 88:11120-11123.)

Antibodies may also be produced by inducing *in vivo* production in the lymphocyte population or by screening immunoglobulin libraries or panels of highly specific binding reagents. (See, *e.g*., Orlandi et al. (1989) Proc Natl Acad Sci 86:3833-3837; Winter and Milstein (1991) Nature 349:293-299.) Antibody fragments that contain specific binding sites for the target polypeptide may also be generated. Such antibody fragments include, but are not limited to, F(ab')₂ fragments, which can be produced by pepsin digestion of the antibody molecule, and Fab fragments, which can be generated by reducing the disulfide bridges of the F(ab')₂ fragments. Alternatively, Fab expression libraries may be constructed to allow rapid and easy identification of monoclonal Fab fragments with the desired specificity. (See, e.g., Huse et al. (1989) Science 254:1275-1281.)

Alternatively, human somatic cells capable of producing antibody, specifically B lymphocytes, are suitable for fusion with myeloma cell lines. B lymphocytes from peripheral blood, or from biopsied spleens, tonsils, or lymph nodes of an individual may be used. In addition, human B cells may be directly immortalized by the Epstein-Barr virus. (Cole et al. (1995) Monoclonal Antibodies and Cancer Therapy, Alan R. Liss, Inc., pp. 77-96.)

Myeloma cell lines suited for use in hybridoma-producing fusion procedures preferably are non-antibody-producing, have high fusion efficiency, and enzyme deficiencies that render them incapable of growing in certain selective media which support the growth of the desired hybridomas. Examples of myeloma cell lines that may be used for the production of fused cell lines that may be in the present invention include P3X63Ag8, P3X63Ag8-653, NS1/1.Ag4.1, Sp210-Ag14, FO, NSO/U, MPC-11, MPC11-X45-GTG 1.7, S194/5XX0 Bul, all derived from mice; R210.RCY3, Y3-Ag 1.2.3, IR983F and 4B210, all derived from rats; and U-266, GM1500-GRG2, LICR-LON-HMy2, UC729-6, all derived from humans. (See, *e.g*., Goding, *supra,* pp. 65-66; Campbell (1984) Monoclonal Antibody Technology: Laboratory Techniques in Biochemistry and Molecular Biology, Vol. 13 (Burden and Von Knippenberg, eds.), Elsevier, Amsterdam, pp. 75-83)

In the present invention, "human" or "humanized" antibodies directed to CTGF or to fragments thereof can also be used. Humanized antibodies are antibodies, or antibody fragments, that have the same or similar binding specificity as a parent antibody, *(e.g.,* of mouse origin) and increased human characteristics. Humanized antibodies may be obtained, for example, by chain shuffling or by using phage display technology. For example, a polypeptide comprising a heavy or light chain variable domain of a non-human antibody specific for CTGF or fragments thereof is combined with a repertoire of human complementary (light or heavy) chain variable domains. Hybrid pairings specific for the antigen of interest are selected. Human chains from the selected pairings may then be combined with a repertoire of human complementary variable domains (heavy or light) and humanized antibody polypeptide dimers can be selected for binding specificity for an antigen. Techniques described for generation of humanized antibodies that can be used in the method of the present invention are disclosed in, for example, U.S. Patent Nos. 5,565,332; 5,585,089; 5,694,761; and 5,693,762. Furthermore, techniques described for the production of human antibodies in transgenic mice are described in, for example, U.S. Patent Nos. 5,545,806 and 5,569,825.

Antibodies produced as described herein can be used to identify CTGF or fragments thereof in, e.g., blood, serum, plasma, urine, vitreous, synovial fluid, cerebral spinal fluid, saliva, biopsies from specific tissues, etc., or other biological samples, cell culture samples, or assay samples. The amount of a particular polypeptide present could be determined, for example, by quantitative image analysis. Alternatively, the mRNA of the target polypeptide could be determined, such as by reverse transcriptase polymerase chain reaction (PCR) using a biological sample. In particular, in this method, mRNA from, for example, a tissue sample in total, or that encodes the target polypeptide or fragments thereof, could be reverse transcribed to DNA and then amplified through PCR using specific primer sequences. Quantitation of mRNA could be determined, for example, by a competition reaction using equal volumes of the patient sample run against a series of decreasing known concentrations, e.g., of a mimic or mutant cDNA fragment.

### CTGF Assays

Preferred assays for detecting N-terminal fragments of CTGF polypeptides are performed using monoclonal or polyclonal antibodies. A variety of assays can be utilized in order to detect antibodies that specifically bind to the desired protein from a sample, or to detect the desired protein bound to one or more antibodies from the sample. Exemplary assays are described in detail in Harlow and Lane, *supra;* Burtis and Ashwood (1999) Tietz Textbook of Clinical Chemistry, 3rd edition, W.B. Saunders, Philadelphia; and Kaplan, Pesce, and Kazmierczak (1996) Clinical Chemistry: Theory, Analysis, Correlation, 3rd edition, Mosby, St. Louis. Representative examples of such assays include: countercurrent immuno-electrophoresis (CIEP) assays, radioimmunoassays, radioimmunoprecipitations, enzyme-linked immunosorbent assays (ELISA), dot blot assays, inhibition or competition assays, sandwich assays, immunostick (dip-stick) assays, simultaneous assays, immunochromatographic assays, immunofiltration assays, latex bead agglutination assays, immunofluorescent assays, biosensor assays, and low-light detection assays (see, e.g., U.S. Patent. Nos. 4,376,110 and 4,486,530; also see, Goding, *supra*).

A fluorescent antibody test (FA-test) uses a fluorescently labeled antibody (e.g., antibody directed to the N-terminal region of CTGF) able to bind to one of the proteins. Detection can be accomplished using fluorescence microscopy and visual determinations, yielding a qualitative result. In one embodiment, this assay is used for the examination of tissue samples or histological sections.

In latex bead agglutination assays, antibodies are conjugated to latex beads. The antibodies conjugated to the latex beads are then contacted with a sample under conditions permitting the antibodies to bind to desired proteins in the sample. Qualitative or quantitative result can be determined.

Enzyme immunoassays (EIA) include a number of different assays able to utilize the antibodies and detect CTGF. For example, a heterogeneous indirect EIA uses a solid phase coupled with an antibody to CTGF and an affinity purified, anti-IgG immunoglobulin preparation. Preferably, the solid phase is a polystyrene microtiter plate. The antibodies and immunoglobulin preparation are then contacted with the sample under conditions permitting antibody binding, which conditions are well known in the art. The results of such an assay can be assessed visually, but are preferably determined using a spectrophotometer, such as an ELISA plate reader, to yield a quantitative result. An alternative solid phase EIA format includes plastic-coated ferrous metal beads able to be moved during the procedures of the assay by means of a magnet. Yet another alternative is a low-light detection immunoassay format. In this highly sensitive format, the light emission produced by appropriately labeled bound antibodies are quantitated automatically. Preferably, the reaction is performed using microtiter plates.

In an alternative embodiment, a radioactive tracer is substituted for the enzyme-mediated detection in an EIA to produce a radioimmunoassay (RIA), using methods known to one of skill in the art.

Enzyme-linked immunosorbent assays (ELISA) can also be performed. In one embodiment, the ELISA comprises the following steps: (1) coating a N-terminal fragment of CTGF of the present invention onto a solid phase; (2) incubating a sample suspected of containing CTGF antibodies with the polypeptide coated onto the solid phase under conditions that allow the formation of an antigen-antibody complex; (3) adding an anti-antibody (such as anti-IgG) conjugated with a label to be captured by the resulting antigen-antibody complex bound to the solid phase; and, (4) measuring the captured label and determining therefrom whether the sample has CTGF antibodies.

In a capture-antibody sandwich enzyme assay, the desired protein is bound between an antibody attached to a solid phase, preferably a polystyrene microtiter plate, and a labeled antibody. Preferably, the results are measured using a spectrophotometer, such as an ELISA plate reader. In a sequential assay format, reagents are allowed to incubate with the capture antibody in a stepwise fashion. The test sample is first incubated with the capture antibody. Following a wash step, incubation with the labeled antibody occurs. In a simultaneous assay, the two incubation periods described in the sequential assay are combined. This eliminates one incubation period plus a wash step.

A dipstick/immunostick format is essentially an immunoassay except that the solid phase, instead of being a polystyrene microtiter plate, is a polystyrene paddle or dipstick. Reagents are the same and the format can either be simultaneous or sequential.

In a chromatographic strip test format, a capture antibody and a labeled antibody are dried onto a chromatographic strip, which is typically nitrocellulose or nylon of high porosity bonded to cellulose acetate. The capture antibody is usually spray dried as a line at one end of the strip. At this end there is an absorbent material that is in contact with the strip. At the other end of the strip the labeled antibody is deposited in a manner that prevents it from being absorbed into the membrane. Usually, the label attached to the antibody is a latex bead or colloidal gold. The assay may be initiated by applying the sample immediately in front of the labeled antibody.

Immuno-filtration/immuno-concentration formats combine a large solid phase surface with directional flow of sample/reagents, which concentrates and accelerates the binding of antigen to antibody. In a preferred format, the test sample is pre-incubated with a labeled antibody then applied to a solid phase such as fiber filters or nitrocellulose membranes or the like. The solid phase can also be pre-coated with latex or glass beads coated with capture antibody. Detection of analyte is the same as standard immunoassay. The flow of sample/reagents can be modulated by either vacuum or the wicking action of an underlying absorbent material.

A threshold biosensor assay is a sensitive, instrumented assay amenable to screening large numbers of samples at low cost. In one embodiment, such an assay comprises the use of light addressable potentiometric sensors wherein the reaction involves the detection of a pH change due to binding of the desired protein by capture antibodies, bridging antibodies and urease-conjugated antibodies. Upon binding, a pH change is effected that is measurable by translation into electrical potential. The assay typically occurs in a very small reaction volume, and is very sensitive. Moreover, the reported detection limit of the assay can be 1,000 molecules of urease per minute.

### Methods of Diagnosis, Prognosis, Prevention, and Treatment

The present invention provides for methods of diagnosing, prognosing, and monitoring therapeutic treatment and efficacy based on detecting and/or measuring the levels of CTGF N-terminal fragments. N-terminal fragments of CTGF are more stable than CTGF or C-terminal fragments of CTGF and can be used to provide more sensitive and accurate detection and quantitation of CTGF and CTGF fragment levels in a sample.

In one aspect, the invention provides a method for diagnosing a CTGF-associated disorder. In one embodiment, the method for diagnosing a CTGF-associated disorder involves obtaining a sample and detecting and quantitating in the sample the levels of N-terminal fragments of CTGF; comparing the levels of fragments of CTGF in the sample to that of a standard amount of fragments of CTGF, wherein an increased or decreased amount of fragments of CTGF in the sample is indicative of the presence of a CTGF-associated disorder. Disorders associated with aberrant (e.g., increased or decreased) levels of CTGF or of fragments of CTGF include, but are not limited to, proliferative disorders and disorders associated with altered expression and deposition of extracellular matrix-associated proteins. Such disorders include, for example, cancers such as breast, prostate, ovarian, colorectal, pancreatic, and gastrointestinal cancer; and atherosclerosis, arthritis, retinopathies such as diabetic retinopathy; nephropathies such as diabetic nephropathy; cardiac, pulmonary, liver, and kidney fibrosis, and diseases associated with chronic inflammation and/or infection. CTGF-associated disorders are also associated with conditions such as myocardial infarction, diabetes, peritoneal dialysis, chronic and acute transplant rejection, chemotherapy, radiation therapy, and surgery. Additional CTGF-associated disorders are described *supra.*

Methods are also provided for identifying whether or not an individual has a predisposition to develop a CTGF-associated disorder. In one embodiment, the method for identifying a predisposition to a CTGF-associated disorder involves obtaining a sample and detecting and quantitating in the sample the levels of N-terminal fragments of CTGF; comparing the levels of fragments of CTGF in the sample to that of a standard amount of fragments of CTGF, wherein an increased or decreased amount of fragments of CTGF in the sample is indicative of the presence of a CTGF-associated disorder.

In another aspect, the invention provides a method for monitoring the progression of a CTGF-associated disorder, the method comprising obtaining a sample; detecting and quantitating the level of N-terminal fragments of CTGF in the sample; and comparing the levels of fragments of CTGF in the sample with a known standard amount of fragments of CTGF, or with a first level of fragments of CTGF in a sample obtained from the same individual or source at an earlier or previous time, or at an earlier stage in the progression of a disease, wherein a difference between the level of fragments of CTGF in the sample and the standard or first level is indicative of the progression of a CTGF-associated disorder.

In another aspect, the invention provides a method for determining the prognosis of the course of a CTGF-associated disorder, the method comprising obtaining a sample; detecting and quantitating the levels of N-terminal fragments of CTGF in the sample; and comparing the level of fragments of CTGF in the sample with that measured in a sample from the individual obtained at a previous time, or at an earlier stage in the progression of a disease, wherein a difference between the level of fragments of CTGF in the sample taken at one time and the level of fragments of CTGF in the sample taken at another time is determinant of the prognosis of the course of a CTGF-associated disorder.

In another aspect, the invention provides a method for monitoring the therapeutic efficacy of treatment of a CTGF-associated disorder, the method involving obtaining a sample from a subject having a CTGF-associated disorder and receiving treatment for that disorder; detecting and quantitating the levels of N-terminal fragments of CTGF in the sample; and comparing the level of fragments of CTGF in the sample with a standard level of fragments of CTGF, wherein a difference between the level of fragments of CTGF in the sample and the standard level is indicative of the therapeutic efficacy of treatment of a CTGF-associated disorder.

In another aspect, the invention provides a method for monitoring the therapeutic efficacy of treatment of a CTGF-associated disorder, the method comprising obtaining a sample; detecting and quantitating the levels of N-terminal fragments of CTGF in the sample; and comparing the level of fragments of CTGF in the sample with that in a sample taken from the individual at a previous time, or in a sample or samples obtained sequentially over time, or at an earlier stage in the progression of a disease, wherein a difference between the level of fragments of CTGF in the sample taken at one time and the level of fragments of CTGF in the sample taken at another time are indicative of the therapeutic efficacy of treatment of a CTGF-associated disorder.

### Compound Screening and Identification

The present disclosure further provides methods of screening for and identifying compounds that decrease or increase the expression, levels, or activity of CTGF or fragments of CTGF (*e.g.,* N-terminal fragments of CTGF, C-terminal fragments of CTGF, etc.).

One method of screening for compounds that increase the expression, level, or activities of CTGF fragments (e.g., N-terminal fragments of CTGF, C-terminal fragments of CTGF, etc.) involves obtaining a sample; contacting the sample with a compound; and detecting and measuring CTGF and CTGF fragment expression, level, or activity in the sample, wherein increased CTGF fragment expression, level, or activity is indicative of a compound having activity that increases CTGF fragment expression, levels, or activity.

In another method of screening for compounds that decrease the expression, level, or activities of CTGF fragments (e.g., N-terminal fragments of CTGF, C-terminal fragments of CTGF), the method involving obtaining a sample; contacting the sample with a compound; and detecting and measuring CTGF and CTGF fragment expression, levels, or activity in the sample, wherein decreased CTGF fragment expression, level, or activity is indicative of a compound having activity that decreases CTGF fragment expression, levels, or activity.

Compounds can be identified by any of a variety of screening techniques known in the art. Such screening methods may allow for CTGF polypeptides or the compounds to be free in solution, affixed to a solid support, borne on a cell surface, associated with extracellular matrix, or located intra-cellularly. For example, microarrays carrying test compounds can be prepared, used, and analyzed using methods available in the art. (See, *e.g.,* Shalon et al. (1995) PCT Application No. WO95/35505; Baldeschweiler et al. (1995) PCT Application No. WO95/251116; Brennan et al. (1995) U.S. Patent No. 5,474,796; Heller et al. (1997) U.S. Patent No. 5, 605,662.)

Various assays and screening techniques can be used to identify small molecules that modulate (*e.g*., increase or decrease) the expression, level, or activity of CTGF or fragments of CTGF (*e.g*., N-terminal fragments of CTGF, C-terminal fragments of CTGF). These methods can also serve to identify antibodies and other compounds that interact with or affect the expression, level, or activity of CTGF or fragments of CTGF, and can therefore be used as drugs and therapeutics in the present methods. (See, *e.g*., Enna et al. (1998) Current Protocols in Pharmacology, John Wiley and Sons.) Assays will typically provide for detectable signals associated with the binding of the compound to a protein or cellular target. Binding can be detected by, for example, fluorophores, enzyme conjugates, and other detectable labels well known in the art. The results may be qualitative or quantitative.

In any of the methods described herein, samples may be obtained from cells in culture, or from a mammal, preferably from a human subject. Samples may be obtained, for example, from conditioned media of cultured cells, or from blood, urine, serum, plasma, vitreous, synovial fluid, cerebral spinal fluid, saliva, amniotic fluid, or obtained from biopsy of tissue. Any one of a variety of methods known in the art can be used to detect and quantitate the levels of CTGF or of fragments thereof. Such methods include, but are not limited to, for example, the use of antibodies directed to N-terminal fragments or C-terminal fragments of CTGF; isolation of N-terminal fragments or C-terminal fragments of CTGF (e.g., using mass spectrometry, liquid chromatography, polyacrylamide gel electrophoresis, western blot analysis, or other conventional chemical analyses, etc.); or any other of the methods and procedures known to one of skill in the art. In certain aspects, the methods involve detection of N-terminal regions/domains of CTGF included within CTGF. In another aspect, the methods comprise measuring the level of N-terminal fragments of CTGF which have been separated (e.g., cleaved) and are distinct from C-terminal regions of CTGF or from CTGF.

These and other embodiments of the present invention will readily occur to those of ordinary skill in the art in view of the disclosure herein.

### EXAMPLES

The invention will be further understood by reference to the following examples, which are intended to be purely exemplary of the invention. These examples are provided solely to illustrate the claimed invention. The present invention is not limited in scope by the exemplified embodiments, which are intended as illustrations of single aspects of the invention only. Any methods which are functionally equivalent are within the scope of the invention. Various modifications of the invention in addition to those described herein will become apparent to those skilled in the art from the foregoing description and accompanying figures.

### Example 1: Production of recombinant human CTGF

Recombinant human CTGF (rhCTGF) was prepared as follows. A full-length human CTGF cDNA (DB60R32) was obtained from Dr. Gary Grotendorst (University of Miami Medical School). (Bradham et al. (1991) J Cell Biol 114:1285-1294.) A CTGF cDNA comprising only the open reading frame was generated by the polymerase chain reaction using DB60R32 DNA as template and the following primers (5'-gctccgcccgcagtgggatccatgaccgccgcc-3' (SEQ ID NO:3) and 5'-ggatccggatcctcatgccatgtctccgta-3') (SEQ ID NO:4), which added BamHI restriction enzyme sites (underlined) to either end of the amplified product. The resulting amplified DNA fragment was digested with BamHI, purified on an agarose gel, and subcloned directly into the BamHI site of the baculovirus (donor) expression plasmid pFastBac1 (Invitrogen, Carlsbad CA). The pFastBacl/CTGF cDNA vector construct was transposed into bacmid DNA and recombinant baculovirus was generated by following the manufacturer's protocol outlined in the BAC-TO-BAC Baculovirus Expression System manual (Invitrogen). Expansion of recombinant baculovirus titers in Sf9 insect cells was performed using standard procedures known in the art. (Murphy and Piwnica-Worms, (1984) Current Protocols in Molecular Biology, Vol. 2 (Ausubel et al., Eds.) John Wiley & Sons, Inc.)

Expression and production of rhCTGF was performed as follows. HIGH FIVE insect cells, adapted to suspension growth, were grown in SF900II medium (Invitrogen) to a cell density of 1.0x10⁶ cells/ml. The cells were then infected with baculovirus containing CTGF at a multiplicity of infection of 10:1. Following infection, the cells were incubated at 27°C for 40 hours. The cells were then pelleted by centrifugation, and the CTGF-containing conditioned medium was collected and filtered through a 0.22 µm filter. The conditioned medium was then directly applied to a 5 ml Hi-Trap heparin column (Amersham Biosciences Corp., Piscataway NJ), which had been pre-equilibrated with 50 mM Tris, pH 7.2. The heparin column was then washed with 100 ml of 350 mM NaCl/50 mM Tris, pH 7.2, and bound CTGF was eluted with a linear gradient of 350 mM NaCl to 1200 mM NaCl over 15 column volumes. Fractions were analyzed for the presence of CTGF by SDS-PAGE.

Fractions containing CTGF were pooled and diluted 1:4 with 50 mM Tris, pH 7.5. The CTGF pool was loaded over a carboxy methyl (CM) ion exchange column (POROS CM column, PerSeptive Biosystems, Framingham, MA), which had been pre-equilibrated with 10 column volumes of 50 mM Tris, pH 7.5. The CM column was washed with 350 mM NaCl/50 mM Tris, pH 7.5, and bound CTGF was eluted with a linear gradient of 350 mM NaCl to 1200 mM NaCl over 15 column volumes. The fractions were analyzed for the presence of CTGF by SDS-PAGE. Fractions containing CTGF were pooled and used as CTGF material.

### Example 2: CTGF N-terminal and C-terminal fragment production

N-terminal fragments and C-terminal fragments of CTGF were prepared as follows. Recombinant human CTGF, prepared and purified as described above, was digested by treatment with chymotrypsin beads (Sigma Chemical Co., St. Louis MO), using 1.5 mg of CTGF per unit of chymotrypsin. Chymotrypsin treatment of rhCTGF was allowed to proceed at room temperature for 6 hours. The digested product and chymotrypsin beads were centrifuged, the chymotrypsin beads were discarded, and the supernatant, containing enyzmatically cleaved rhCTGF, was diluted 1:5 with 50 mM Tris, pH 7.5. The diluted supernatant was applied to a Hi-Trap heparin column. The flow-through was collected, and contained N-terminal fragments of CTGF. The heparin column was washed with 350 mM NaCl, and bound C-terminal fragments of CTGF and undigested CTGF were eluted with a linear gradient of 350 mM to 1200 mM NaCl, as described above. The fractions were analyzed for the presence of C-terminal fragments of CTGF by SDS-PAGE. Fractions containing C-terminal fragments of CTGF were pooled according to the observed purity of C-terminal CTGF.

The heparin column flow-through, which contained N-terminal fragments of CTGF, was adjusted to contain 0.5 M ammonium sulfate /50 mM Tris, pH 7.5. The sample containing N-terminal fragments of CTGF was then loaded onto a 15 ml phenyl sepharose HP column (Amersham Pharmacia Biotech), which had been pre-equilibrated with 0.5 M ammonium sulfate /50 mM Tris, pH 7.5. The phenyl sepharose HP column was then washed with 15 column volumes of 0.5 M ammonium sulfate/ 50 mM Tris, pH 7.5. Bound N-terminal fragments of CTGF were eluted with a linear gradient of 0.5 M to 0 M ammonium sulfate /50 mM Tris, pH 7.5, over approximately 15 column volumes. Fractions were analyzed for the presence of N-terminal fragments of CTGF by SDS-PAGE. Fractions containing N-terminal fragments of CTGF were pooled. This pool, containing N-terminal fragments of CTGF, was concentrated and the buffer exchanged with 50 mM Tris, 400 mM NaCl, pH 7.2, using Amicon ultrafiltration, YM10 membrane.

### Example 3: Production of antibodies against CTGF and fragments of CTGF

Monoclonal and polyclonal antibodies were prepared against purified rhCTGF. Recombinant human CTGF, N-terminal fragments of CTGF, and C-terminal fragments of CTGF were produced and purified as described above. Monoclonal and polyclonal antibodies specific to human CTGF or fragments of CTGF were made by standard methods. (See, *e.g.*, Goding, *supra;* and Schook, *supra*.)

Monoclonal antibodies to human CTGF are generally prepared as follows. Healthy adult mice are immunized subcutaneously with 50 to 100 µg of CTGF, N-terminal fragments of CTGF, or C-terminal fragments of CTGF. After six to ten weeks, mice are boosted with additional antigen formulated in incomplete Freund's adjuvant. Approximately one week after each booster injection, the serum from immunized mice is evaluated for the presence of antibodies to CTGF, as determined by ELISA analysis. Mice producing the greatest antibody responses (i.e., highest titer) to CTGF or fragments thereof are chosen for the production of hybridomas. Isolated spleen cells from these mice are fused to a mouse myeloma partner, such as, for example, P3X63Ag8-653, using polyethylene glycol, following procedures well know in the art. Hybridomas producing antibody reactive to CTGF are identified by ELISA and subsequently cloned by limiting dilution at least two times.

Alternatively, human monoclonal antibodies to human CTGF were made by the following method. Cohorts of HUMAB mice (Medarex, Inc., Princeton NJ) were immunized with different CTGF immunogens including a) purified rhCTGF produced in baculovirus, b) N- and C-terminal fragment halves of CTGF generated by chymotrypsin cleavage of whole rhCTGF, and c) various synthetic CTGF peptides. HUMAB mice (Medarex) are genetically engineered transgenic mice that respond to immunogens by making fully human antibodies. Hybridomas generated from 25 splenic fusions were screened and approximately 140 clonally selected monoclonal antibody-producing cell lines were characterized. The affinity of each antibody population was measured using RIA, wherein whole rhCTGF was radio-iodinated and added to wells containing immobilized monoclonal antibody and varying quantities of unlabelled CTGF.

### Example 4: Characterization of antibodies

Hybridomas producing antibodies to human CTGF were prepared as described in Example 3. Cloned hybridoma cells were grown in Dulbecco's Modified Eagle Medium-High Glucose/RPMI 1640 (50:50) with 8 mM L-Glutamine, ½ x Nonessential Amino Acids, and 10% Fetal Bovine Serum. Cells expanded for antibody preparation were grown in the same media with 1.5% Low IgG Fetal Bovine Serum for 4-9 days at 37°C and 6% CO₂. The resulting conditioned media was cleared of cells and concentrated using a tangential flow filtering/concentrating system. The concentrate was passed over a protein-A column and bound monoclonal antibodies eluted with 100 mM glycine, pH 3. The eluate was neutralized with 1 M Tris, pH 8.0, and dialyzed against PBS.

Each resulting monoclonal antibody obtained was mapped and assigned to specific regions of CTGF to which it bound, using standard binding and blocking experiments. (Harlow and Lane, *supra;* Burtis and Ashwood, *supra;* and Kaplan, Pesce, and Kazmierczak, *supra,* Chapter 10 (Immunochemical Techniques) and Chapter 11 (Competitive Binding Assays).) For example, antibodies of the same domain or epitope specificity would block binding of any other antibody of the same domain or epitope specificity, particularly if added in a sequential manner. More refined mapping of the epitopes on CTGF to which the monoclonal antibodies bound was performed by ELISA analysis using specific fragments of recombinantly expressed CTGF. For example, monoclonal antibodies which recognized N-terminal domains or regions of CTGF were identified by ELISA analysis against immobilized recombinantly expressed exon 2 of CTGF. Monoclonal antibodies were mapped to recombinantly expressed domains of CTGF, such as those encoded by exon 2, exon 3, exon 4, or exon 5 of CTGF polynucleotide sequence.

In this manner, antibodies that specifically recognize N-terminal domains or N-terminal fragments of CTGF (e.g., N1, an antibody having specificity for a first N-terminal fragment epitope or epitopes; and N2, an antibody having specificity for a second N-terminal fragment epitope or epitopes), or C-terminal domains or C-terminal fragments of CTGF (*e.g.,* C-terminal fragment epitopes C1, C2, C4, C5, or CK) were selected and characterized. (See, e.g., Figures 1A and 1B.)

### Example 5: Assay for CTGF and fragments of CTGF

Immunoassays were developed to detect and accurately quantitate CTGF polypeptides. Specifically, methods were developed to detect and quantitate CTGF (referred to as the N-C assay), N-terminal fragments of CTGF (referred to as the N-N assay), and C-terminal fragments of CTGF (referred to as the C-C assay). Figure 1C illustrates the different sandwich assay formats.

### CTGF

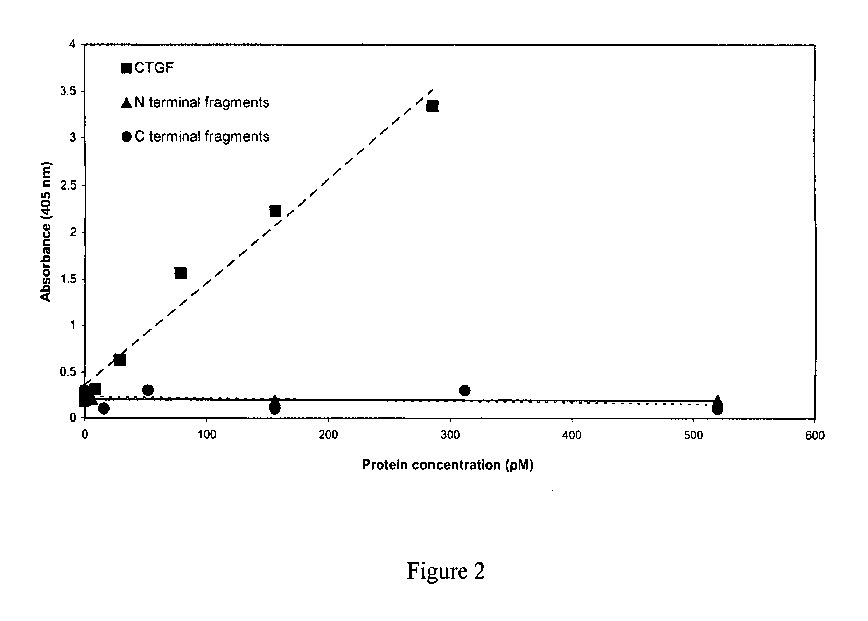

Whole CTGF was detected and quantitated using one antibody that recognized an N-terminal domain of CTGF (e.g., N1 and N2 as described above and shown in Figure 1A) and a second antibody that recognized a C-terminal domain of CTGF (e.g., C1-C5 as described above and shown in Figure 1A). This assay is referred to as the N-C sandwich assay (Figure 1C). The N-terminal domain specific antibody (e.g., monoclonal antibody (mAb) 19, which binds to N2) was used to capture CTGF, while the C-terminal domain specific antibody (e.g., mAb 25, which binds to C4) was used to detect the captured CTGF polypeptide. Alternatively, an antibody that recognizes a C-terminal domain of CTGF or heparin optionally linked to a carrier, e.g., BSA, could be used to capture CTGF, and an antibody that recognizes an N-terminal domain of CTGF could be used to detect the captured CTGF polypeptide. In either method, the use of two reagents, one that recognizes an N-terminal domain of CTGF and the other that recognizes a C-terminal domain of CTGF, allowed for the detection of CTGF, but not of N-terminal fragments of CTGF or C-terminal fragments of CTGF (Figure 2).

### N-terminal fragments of CTGF

N-terminal fragments of CTGF were detected and quantitated using two antibodies that recognize N-terminal domains of CTGF (e.g., N1 and N2 as described above and shown in Figure 1A). This assay is referred to as the N-N assay (Figure 1C). One of the N-terminal domain specific antibodies served as a capture antibody (e.g., mAb 19, which binds to N2), while the other served as a detection antibody (e.g., mAb 6, which binds to N1). CTGF was also detected using the N-N assay, as CTGF contains N-terminal domains of CTGF, which are recognized by the antibodies employed.

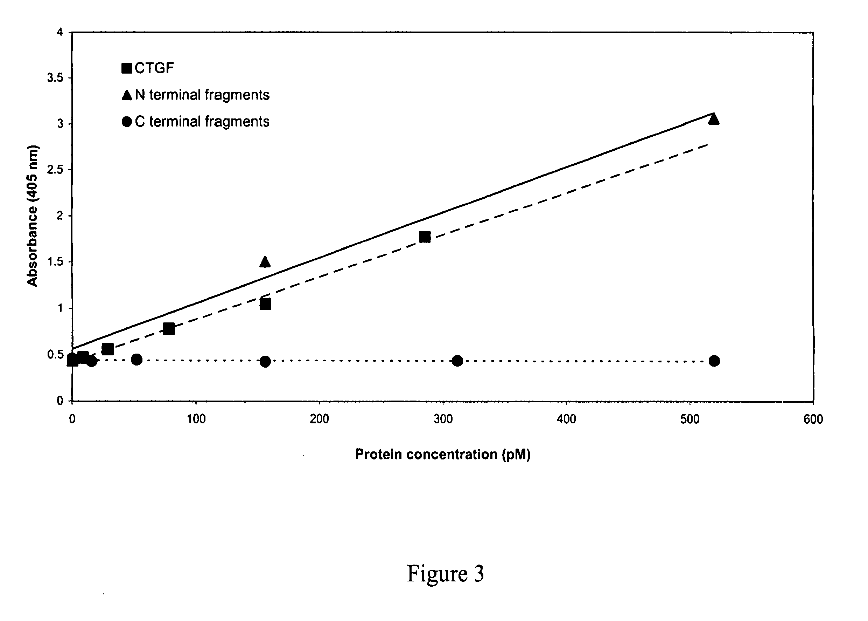

To determine the amount of N-terminal fragments of CTGF in a sample, the level of CTGF in the sample was determined (using the N-C assay, *supra*), and the level of N-terminal fragments of CTGF in the sample was determined (using the N-N assay). The amount of CTGF determined was then subtracted from the amount of N-terminal fragments determined, resulting in the level of N-terminal fragments of CTGF present in the sample. Unless otherwise indicated, data obtained using the N-N assay, which detected and quantitated the levels of both CTGF and N-terminal fragments of CTGF (Figure 3), have been adjusted to show the specific levels of N-terminal fragments of CTGF in each sample (i.e., the levels of CTGF in a sample have been subtracted from the values obtained in that sample using the N-N assay). Alternatively, samples can be cleared of CTGF using a heparin affinity column prior to conducting the N-N assay.

### C-terminal fragments of CTGF

C-terminal fragments of CTGF were detected and quantitated using two antibodies that recognize C-terminal domains of CTGF (*e.g.*, C1 and C4 as described above and shown in Figure 1A). This assay is referred to as the C-C assay (Figure 1C). One of the C-terminal domain specific antibodies served as a capture antibody (*e.g*., mAb 25, which binds to C4), while the other served as a detection antibody (*e.g*., mAb 51, which binds to C1). Alternatively, heparin linked to a carrier, *e.g*., BSA, could be used to capture C-terminal domains of CTGF. CTGF was also detected in this assay, as CTGF contains C-terminal domains of CTGF.

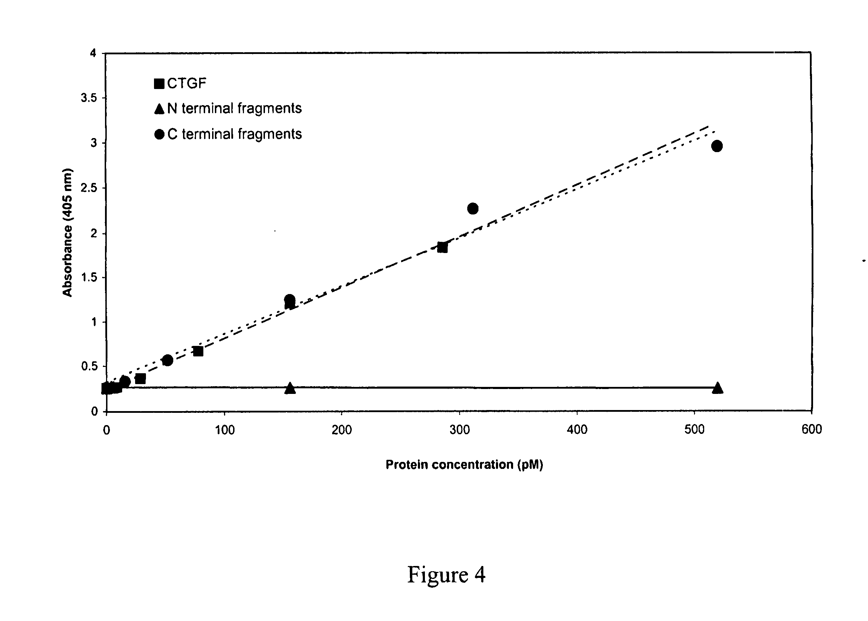

To determine the amount of C-terminal fragments of CTGF in a sample, therefore, the level of CTGF in the sample was determined (using the N-C assay), and the level of C-terminal fragments of CTGF in the sample was determined (using the C-C assay). The amount of CTGF determined was then subtracted from the amount of C-terminal fragments determined, resulting in the level of C-terminal fragment present in the sample. Unless otherwise indicated, data obtained using the C-C assay, which detected and quantitated the levels of both CTGF and C-terminal fragments of CTGF (Figure 4), have been adjusted to show the specific levels of C-terminal fragments of CTGF in each sample (i.e., the levels of CTGF in a sample have been subtracted from the values obtained in that sample using the C-C assay). Alternatively, samples can be cleared of CTGF using an N-terminal fragment specific reagent, *e.g.,* an antibody, prior to conducting the C-C assay.

Quantitation of the levels of CTGF, N-terminal fragments of CTGF, and C-terminal fragments of CTGF in samples were determined by extrapolating the absorbance values obtained with each ELISA assay described (*i*.*e*., N-C assay, detecting CTGF; N-N assay, detecting CTGF plus N-terminal fragments of CTGF; and, C-C assay, detecting CTGF plus C-terminal fragments of CTGF) to that obtained using CTGF standards of known concentrations. The data were analyzed by quadratic non-linear regression (SOFTMAX PRO software, Molecular Devices, Sunnyvale, CA), providing a best-fit equation describing the standard curve and relating absorbance to CTGF concentration on a molar basis.

### Example 6: CTGF and CTGF fragment detection and quantitation

### Low-protein samples

Procedures for performing CTGF assays were described above in Example 5. Modifications to these methods could be made for detecting and quantitating CTGF and fragments of CTGF in various samples or sample types. For example, to detect and quantitate human CTGF and fragments of CTGF in low-protein samples (*e.g.*, urine, synovial fluids, peritoneal dialysis fluids, CSF fluid, saliva, cell culture media), a sandwich ELISA using anti-human CTGF monoclonal antibodies was developed. The ELISA assay was set up as follows. The wells of a microtiter plate were first coated with a capture antibody which recognizes a region or domain of CTGF. For use in the ELISA, the capture antibody was diluted to a final concentration of 10 µg/ml in 50 mM sodium bicarbonate, pH 8.5, and fifty microliters of the diluted capture antibody solution was added to each well of a 96-well MAXISORB microtiter plate (Nalge Nunc International, Rochester NY). The plate was then covered and stored at 2 to 8°C overnight to allow antibody binding to the plate. The capture antibody solution was removed from the plates, and the unbound sites in each well were then blocked with 150 µl of blocking buffer, containing 1% BSA in calcium-free and magnesium-free PBS with 0.05% sodium azide. The plate was then covered and stored at 2 to 8°C for 1 to 14 days. Immediately prior to use in a CTGF detection and quantitation assay, the blocking buffer was removed from the wells, and the wells of each plate were washed once with wash buffer (calcium-free and magnesium-free PBS containing 0.1% Tween 20).

CTGF standard solutions for use in the ELISA assays were prepared as follows. A frozen aliquot of rhCTGF, prepared as described above, was thawed and diluted to a final concentration of 1.1 µg/ml in heparin assay buffer (0.45 µm filtered), which contained calcium-free and magnesium-free PBS, 50 mM Tris (pH 7.8), 1 mg/ml BSA, 4 mM MgCl₂, 0.2 mM ZnCl₂, 0.05% sodium azide, 50 µg/ml sodium heparin, and 0.1% TRITON X-100 detergent. The rhCTGF standard stock solution was stored aliquoted at -70°C. Dilutions of the rhCTGF stock solution were then prepared to obtain a range of rhCTGF standard concentrations from 0 ng/ml to 15 ng/ml, diluted in heparin assay buffer. In all ELISA assays described, levels of CTGF, N-terminal fragments of CTGF, and C-terminal fragments of CTGF were determined based on comparison to a CTGF standard.

Biological samples were diluted in heparin assay buffer (typically, dilutions were between 1:2 and 1:20, depending on the particular sample type), and 50 µl of the diluted samples were applied to the coated and blocked microtiter wells, in duplicate or triplicate.

For detection of captured CTGF or of fragments of CTGF, a biotinylated monoclonal antibody of a different CTGF epitope specificity from the coating or capture antibody to CTGF was employed. The biotinylated antibody was added to the above samples and CTGF standards within the microtiter plate wells, such that the CTGF-containing samples and CTGF standards, and the biotinylated anti-CTGF antibody, were incubated concurrently in the capture antibody-coated microtiter plate. Monoclonal antibodies used for detection were biotinylated using biotin:DNP reagent, following the protocol as described by the manufacturer (Molecular Probes, Eugene, OR). A biotin:mAb ratio of 5:1 to 12:1 was typically acceptable. Fifty microliters of biotinylated monoclonal antibody (1000 ng/ml) were added to each well, which already contained samples as described above. The plate was then covered and incubated at 2 to 8°C on a plate rotator for 90 minutes at 100 to 130 rpm.

Afterward, the plates were washed three times with wash buffer. The wells were then incubated with 50 µl of streptavidin-alkaline phosphatase conjugate (Jackson ImmunoResearch Labs, West Grove PA), which was diluted in assay buffer (typically 1:10,000) to a concentration empirically determined for each conjugate lot, based on development of a desired level of signal for the CTGF standard curve. The plate was covered and incubated at 2 to 8°C on a plate rotator for 90 minutes at 100 to 130 rpm. Afterward, the plate was washed three times as described above, and 100 µl of alkaline phosphatase substrate (Sigma Chemical Co.) was added to each well. Color development was monitored visually and with a microtiter plate reader. Once the standard and sample color development reached optimal signal levels, further color development was stopped by the addition of 50 µl of 4 N sodium hydroxide to each well. The microtiter plate was then placed in a microplate reader, and the color development determined by measurement of absorbance units at a wavelength of 405 nm.

### High-protein samples

The following modifications to the CTGF assays described above and in Example 5 were performed when measuring CTGF levels in high protein-containing samples (e.g., blood, serum, plasma, etc.). In order to provide consistency in the concentration or levels of proteins within the standards and samples used in this assay detecting CTGF and CTGF fragments in high-protein samples, heparin assay buffer was supplemented with 10% rat serum in which CTGF was removed.

CTGF was removed from rat serum for use in this assay as follows. Rat serum was incubated overnight with 2% (v/v) heparin-agarose beads, which removed CTGF and C-terminal fragments of CTGF from the rat serum. N-terminal fragments of CTGF were not removed from rat serum by this method due to the fact that N-terminal fragments of CTGF do not bind to heparin. The N-terminal fragments, which remained present in the heparin-agarose treated rat serum, did not interfere with the subsequent CTGF assay, as the assays used for detecting human CTGF did not detect N-terminal fragments of rat CTGF. Alternatively, an antibody affinity column may be used to remove CTGF and fragments of CTGF from mammalian serum in order to create serum in which CTGF and fragments of CTGF were removed.

CTGF standard solutions, prepared as described above, were spiked with 10% (v/v) of the rat serum treated with heparin-agarose beads, as described above. The biotinylated anti-CTGF antibodies (used for detection), diluted in heparin assay buffer, were also spiked with 10% of the rat serum treated with heparin-agarose beads. High-protein containing samples were diluted 1:10 with heparin assay buffer; therefore, all solutions (i.e., CTGF standards, biological samples, etc.) used in the assay contained serum at a final concentration of 10% (v/v). Sandwich ELISA assays described above were then used to detect and quantitate the levels of CTGF and of CTGF fragments in the diluted high-protein containing samples.

### Example 7: CTGF assay specificity

The specificity and reliability of the CTGF ELISA assays described above in Example 5 and Example 6 were examined. Table 1 shows the results of ELISA assays performed with rhCTGF standard using the assays described above for detecting and quantitating CTGF (the N-C assay), N-terminal fragments of CTGF (the N-N assay), and C-terminal fragments of CTGF (the C-C assay).

Assay results presented in Table 1 are absorbances at 405 nm as measured by an ELISA plate reader. Various concentrations of rhCTGF (0 to 11 ng/ml) were used to establish standard curves for each CTGF ELISA assay. The assays were performed in triplicate for each concentration of CTGF, and the statistical mean and coefficient of variation (% CV) of the absorbances for the ELISA assays are included in Table 1.

**Table 1: Representative Standard Curves for CTGF ELISA Assays**

| CTGF Concentration | N-C Assay | %CV | N-N Assay | %CV | C-C Assay | %CV |
|---|---|---|---|---|---|---|
| 0.0 ng/ml | 0.383 | 4.6% | 0.480 | 0.6% | 0.299 | 1.9% |
| 0.33 ng/ml | 0.440 | 5.8% | 0.579 | 3.4% | 0.315 | 2.6% |
| 1.1 ng/ml | 0.611 | 0.5% | 0.758 | 6.1% | 0.379 | 3.6% |
| 3.0 ng/ml | 1.130 | 1.3% | 1.217 | 5.6% | 0.623 | 2.4% |
| 6.6 ng/ml | 1.975 | 1.1% | 2.126 | 4.2% | 1.193 | 3.0% |
| 11.0 ng/ml | 2.817 | 0.9% | 3.109 | 5.0% | 2.091 | 6.9% |

The assays were performed according to the protocols described above in Example 5 and Example 6. Assay specificity was controlled by choice of the capture and labeled (detection) antibody used in each assay. The results showed that the CTGF ELISA assays, for CTGF, CTGF N-terminal fragments, and CTGF C-terminal fragments, as performed in this example, had the sensitivity to detect CTGF polypeptides at concentrations below 1 ng/ml. Additionally, the coefficient of variance (CV) of the assays at a given amount of CTGF was found to be normally in the range of approximately 0 to 6%.

The specificity of each CTGF ELISA assay was examined further. Baculovirus-derived rhCTGF was produced and purified as described above. N-terminal fragments and C-terminal fragments of CTGF were generated by chymotrypsin cleavage of rhCTGF and purified as described above in Example 2. CTGF ELISA assays, which detect and quantitate CTGF (the N-C assay), N-terminal fragments of CTGF (the N-N assay), and C-terminal fragments of CTGF (the C-C assay), were performed using these CTGF polypeptides. The results are shown in Figure 2 (N-C assay), Figure 3 (N-N assay), and Figure 4 (C-C assay).

As indicated above and as shown in Figures 2, 3, and 4, all three CTGF assays described detected CTGF (solid squares in Figures 2, 3, and 4). The CTGF assay only detects CTGF (i.e., containing both N-terminal domains and C-terminal domains of CTGF). The CTGF assay does not detect N-terminal fragments of CTGF (solid triangles in Figures 2, 3, and 4) or C-terminal fragments of CTGF (solid circles in Figures 2, 3, and 4). The N-terminal domain assay detects both CTGF (solid squares in Figure 3) and N-terminal fragments of CTGF (solid triangles in Figure 3), but does not detect C-terminal fragments of CTGF (solid circles in Figure 3). The C-terminal domain assay detects both CTGF (solid squares in Figure 4) and C-terminal fragments of CTGF (solid circles in Figure 4), but does not detect N-terminal fragments of CTGF (solid triangles in Figure 4).

The CTGF and CTGF fragment assays of the present invention were specific to CTGF polypeptides. The structurally related CCN family members NOV and CYR61 (using concentrations as high as 0.5 mg/ml) were not detected using the CTGF assays as described (data not shown).

### Example 8: CTGF detection in the absence or presence of heparin

CTGF is a heparin-binding growth factor, by virtue of heparin binding domains located in the C-terminal region of CTGF. CTGF stability, detection, and recovery were measured and assessed in the presence and absence of heparin at various storage temperatures. Recombinant human CTGF (10 ng/ml) was added to assay buffer, either in the presence or absence of heparin (from Porcine, Sigma Chemical Co.; added to a final concentration of 10 µg/ml). The CTGF-containing samples were then incubated for ten days at the following temperatures: -80°C, -20°C, 4°C, or 25°C.

The amount of CTGF remaining in the sample was then determined by ELISA assay as described above in Example 5. The results of CTGF detection in the absence or presence of heparin are shown below in Table 2.

**Table 2: The Effect of Heparin on CTGF Detection**

| Temperature | -80°C | -20°C | +4°C | +25°C |
|---|---|---|---|---|
| + heparin | 8.9 ng/ml | 9.1 ng/ml | 5.3 ng/ml | 5.3 ng/ml |
| % recovery | 89 % | 91 % | 53 % | 53 % |
| - heparin | 2.7 ng/ml | 5.0 ng/ml | 1.0 ng/ml | 0.8 ng/ml |
| % recovery | 27 % | 50 % | 10 % | 8 % |

As indicated in Table 2, in the absence of heparin, little CTGF was detected, even when the samples were stored at -80°C. After ten days at either 4°C or 25°C in the absence of heparin, approximately 1 ng/ml of the original 10 ng/ml CTGF added to the sample was detected. This indicated that CTGF polypeptide was unstable or perhaps sticky, especially when incubated at temperatures above freezing. When CTGF was incubated in the presence of heparin, detectable CTGF levels after ten days at either 4°C or 25°C were approximately 5 ng/ml, which corresponded to approximately half of the original amount of CTGF added to the assay buffer, and about five-times the level obtained in the absence of heparin. The addition of heparin improved the recovery of CTGF at all temperatures examined. This suggested that heparin provided a stabilizing effect on CTGF, maintained CTGF solubility in aqueous solutions, or protected CTGF from proteolysis, adsorption to plastic, or binding to other macromolecules in solution.

### Example 9: Detection and stability of CTGF and CTGF fragments in cell culture conditioned media

MG-63 cells, a human osteosarcoma cell line, are known to produce and secrete CTGF. Additionally, TGFβ stimulates CTGF expression in these cells. MG-63 cells were obtained from ATCC (CRL-1427). The cells were plated in 12-well tissue culture plates (27,000 cells per well) in Minimal Essential Medium (MEM) containing 10% fetal bovine serum, glutamine, penicillin, streptomycin, and non-essential amino acids. After 48 hrs, the media was removed and replaced with fresh media lacking fetal bovine serum and containing ascorbate phosphate (30 µg/ml) and bovine serum albumin (1 mg/ml). After an additional 24 hrs, the media was replaced with fresh media (MEM lacking fetal bovine serum and containing ascorbate phosphate (30 µg/ml) and bovine serum albumin (1 mg/ml)), containing heparin (10 µg/ml), with or without TGFβ (5 ng/ml). Conditioned media was then harvested from the cells after 0, 24, 92, and 120 hrs and analyzed for CTGF and CTGF fragment levels. Results of the levels of CTGF, N-terminal fragments of CTGF, and C-terminal fragments of CTGF in MG-63 cell conditioned media are shown in Table 3 below.

**Table 3: CTGF and CTGF Fragment Levels in Cell Culture Conditioned Media**

| **ELISA +/- TGFβ** | **24 hours** | **92 hours** | **120 hours** |
|---|---|---|---|
| CTGF (-TGFβ) | 40 ng/ml | 55 ng/ml | 35 ng/ml |
| CTGF (+TGFβ) | 150 ng/ml | 33 ng/ml | 26 ng/ml |
| N-terminal fragments (-TGFβ) | 157 ng/ml | >401 ng/ml | >498 ng/ml |
| N-terminal fragments (+CTGFβ) | 181 ng/ml | >507 ng/ml | >534 ng/ml |
| C-terminal fragments (-TGFβ) | 7 ng/ml | Non detected | Non detected |
| C-terminal fragments (+TGFβ) | Non detected | 1 ng/ml | Non detected |

As shown in Table 3, after 24 hrs of TGFβ treatment, the levels of CTGF detected in the conditioned media of MG-63 cells increased from 40 ng/ml to 150 ng/ml. Following 92 or 120 hrs of TGFβ treatment, however, the levels of CTGF detected in the conditioned media of the cells was greatly reduced from that observed after 24 hrs of treatment.

Similar results were observed for the levels of C-terminal fragments of CTGF. After 24 hrs in the presence of TGFβ, the levels of C-terminal fragments of CTGF in the conditioned media of the cells decreased from 7 ng/ml to levels that were undetectable. Following 92 or 120 hrs of TGFβ treatment, the levels of C-terminal fragments of CTGF in the conditioned media remained low or undetectable. Therefore, over time, the detectable levels of CTGF and C-terminal fragments of CTGF in the conditioned media of MG-63 cells declined, or became undetectable, despite stimulation of CTGF expression with TGFβ.

In contrast, detection and quantitation of N-terminal fragments of CTGF showed that N-terminal fragments of CTGF accumulated in the conditioned media of MG-63 cells over time. The accumulation of N-terminal fragments in the conditioned media was seen both in the presence or absence of TGFβ stimulation of CTGF expression. In spite of the observations that the levels of CTGF increased approximately 4-fold following a 24 hr TGFβ treatment (compared to non-treated cells), only a slight increase in the levels of N-terminal fragments of CTGF was observed in cells treated with TGFβ compared to non-stimulated cells (compare 157 ng/ml with no TGFβ treatment to 181 ng/ml with TGFβ treatment). In this particular situation, measurement of the levels of N-terminal fragments of CTGF alone would not have indicated any increase in CTGF levels following a 24 hr treatment with TGFβ. The ability to detect and distinguish the levels of CTGF, N-terminal fragments of CTGF, and C-terminal fragments of CTGF, therefore, allowed for a sensitive and reliable means to determine the presence and levels of CTGF and fragments thereof, as well as any changes in the expression of CTGF and CTGF fragments under different conditions.

These results indicated that in certain conditions, N-terminal fragments of CTGF could accumulate over time. Accumulation of N-terminal fragments, which coincided with the decline in detectable levels of CTGF, suggested that N-terminal fragments of CTGF are stable (i.e., less susceptible or resistant to proteolysis, clearance, elimination, or adsorption) in biological fluids, such as cell conditioned media, blood, and urine; and therefore, can provide a detectable and stable indicator of CTGF expression.

Additionally, this data suggested that, in certain conditions, CTGF and C-terminal fragments of CTGF could be lost by degradation, excretion, or tissue removal. This data also showed the value and benefit of detecting and distinguishing CTGF, N-terminal fragments of CTGF, and C-terminal fragments of CTGF in cell culture media (as well as in other biological fluids) in determining the levels of CTGF expression and the degree or extent of CTGF cleavage, processing, elimination, clearance, etc.

### Example 10: Western blot analysis of CTGF and CTGF fragments

MG-63 cells were cultured in Dulbecco's Modification of Eagle's Medium (CELLGRO culture medium; Mediatech, Herndon VA) containing 50 µg/ml sodium heparin (Sigma Chemical Co.), 50 µg/ml bovine serum albumin (Sigma Chemical Co.), and without serum. At day one, two, three, and six, conditioned media from the cells were collected, centrifuged at 1200 rpm for 5 min to pellet cell debris, and the supernatants stored frozen.

Analysis of CTGF levels was carried out as follows. N-terminal fragments of CTGF were isolated from the conditioned media by immunoprecipitation with a monoclonal antibody directed against N-terminal domains of CTGF. Immunoprecipitations were performed by incubating the MG-63 cell conditioned media with a monoclonal antibody specific to N-terminal regions of CTGF at a concentration of 10 µg/ml for 1 hour. A 2% (w/w) suspension of protein-A (Sigma Chemical Co.) was added and the sample was incubated for an additional 1 hour. Precipitation with protein-A alone, without the inclusion of any CTGF-specific antibody, was used as a control for non-specific binding. The immunoprecipitation/protein-A complexes were washed twice with buffer containing 150 mM NaCl, 1% TRITON X-100 detergent, 1% deoxycholic acid sodium salt, 0.1% SDS, 50 mM Tris (pH 7.5), and 2 mM EDTA. The samples were then solubilized with Tris-Glycine SDS sample buffer (2X) (Invitrogen) in preparation for electrophoretic separation of precipitated proteins. CTGF and C-terminal fragments of CTGF were isolated from the conditioned media by heparin-sepharose precipitation. Precipitations with heparin-sepharose (Amersham Pharmacia Biotech) were carried out by mixing cell culture conditioned media with 2 % heparin-sepharose suspension (1:1 (w/w) with PBS) for 2 hours at 4°C. The precipitation complexes were washed twice with PBS, and subsequently solubilized as described above.

Following electrophoresis (4-20% Tris-Glycine SDS-PAGE gels (Invitrogen)), the samples were transferred onto nitrocellulose membranes (Invitrogen) for western blot analysis. The membranes were then blocked with 5% skim milk in buffer containing 24.8 mM TRIS base, 2.7 mM potassium chloride, 137 mM sodium chloride, 0.05% Tween 20, pH 7.4.

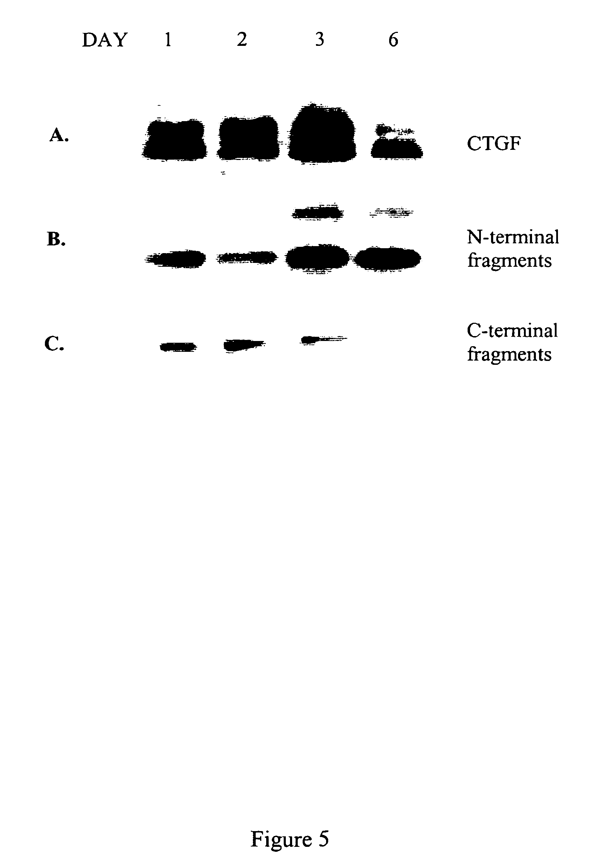

CTGF and fragments of CTGF in the conditioned media of MG-63 cells were detected by western blot analysis, the results of which are shown in Figures 5A, 5B, and 5C. Membranes containing CTGF (Figure 5A) and C-terminal fragments of CTGF (Figure 5C), which had been precipitated with heparin-sepharose beads, were probed with a monoclonal antibody which recognizes C-terminal domains of CTGF, followed by detection with anti-human IgG conjugated with horse radish peroxidase (Jackson ImmunoReasearch). Membranes containing N-terminal fragments of CTGF (Figure 5B), which had been immunoprecipitated with anti-CTGF antibodies, were probed with anti-CTGF chicken polyclonal antibody, and detected with rabbit anti-chicken IgY conjugated with horse radish peroxidase (Zymed, South San Francisco CA). Western blot development was visualized using SUPERSIGNAL chemiluminescent reagent (Pierce Chemical Co., Rockford IL).

Quantitation of the relative changes in the levels of CTGF and fragments of CTGF observed in the conditioned media of MG-63 cells was performed by densitometry of western blots described above. The results of densitometry of the western blot showing CTGF and CTGF fragments are shown in Table 4 below.

**Table 4: Changes in CTGF and CTGF Fragment Levels in Cell Culture Conditioned Media**

| | **Day** 1 | **Day 2** | **Day 3** | **Day 6** |
|---|---|---|---|---|
| CTGF | 1 | 1.17 | 1.99 | 0.43 |
| N-terminal fragment | 1 | 0.4 | 3.03 | 2.35 |
| C-terminal fragment | 1 | 1.08 | 0.53 | 0 |

Detection and quantitation of CTGF, N-terminal fragments of CTGF, and C-terminal fragments of CTGF were demonstrated by western blot analysis. As shown in Figure 5A, CTGF accumulated in the conditioned media of MG-63 cells, reaching a maximum level at approximately day three. At day six of culture, the levels of CTGF in the conditioned media of MG-63 cells were less than half of that determined after the first day of culture, and were only about 20% of the levels determined at day three, which had the highest levels of CTGF measured. This suggested that CTGF was unstable in MG-63 cell conditioned media. The level of N-terminal fragments of CTGF was observed to accumulate in the conditioned media of MG-63 cells with time (Figure 5B). The levels of C-terminal fragments of CTGF did not accumulate in the conditioned media of MG-63 cells; rather, in day six conditioned media, C-terminal fragments of CTGF were undetectable by western blot analysis (Figure 5C).

These observations were consistent with the results obtained by ELISA assay, shown above in Example 9. The decline in detectable levels of CTGF and C-terminal fragments of CTGF suggested that CTGF and C-terminal fragments of CTGF were less stable than N-terminal fragments of CTGF, and therefore may not provide a reliable indicator of CTGF expression or levels in a sample at particular times. Accumulation ofN-terminal fragments of CTGF suggested that N-terminal fragments of CTGF are more stable (i.e., less susceptible or resistant to proteolysis, clearance, elimination, or adsorption) than CTGF or C-terminal fragments of CTGF in biological fluids, such as, for example, cell culture conditioned media. Therefore, N-terminal fragments of CTGF provided a detectable and stable indicator of CTGF expression as measured by western blot analysis. The data also showed the value and benefit of detecting and distinguishing CTGF, N-terminal fragments of CTGF, and C-terminal fragments of CTGF in cell culture media (as well as in other biological fluids) in determining the levels of CTGF expression and the degree or extent of CTGF cleavage, processing, elimination, clearance, etc.

### Example 11: Stability of CTGF and CTGF fragments added to urine

Using methods similar to those described above in Example 8, human urine samples from healthy individuals, to which 10 ng/ml rhCTGF was added, were analyzed by detecting and quantitating the levels of CTGF and of CTGF fragments. For these experiments, CTGF detection and quantitation were performed using the CTGF ELISA assays as described above in Example 5 and Example 6. The normal concentration of N-terminal fragments of CTGF in human urine was previously determined to be approximately 1 to 2 ng/ml.

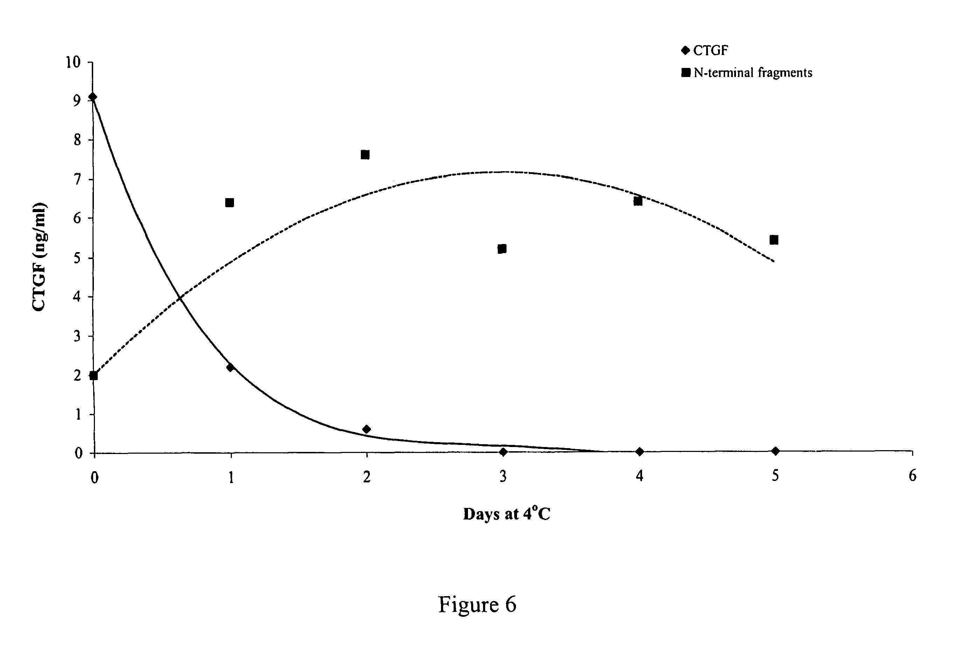

Figure 6 shows levels of CTGF and N-terminal fragments of CTGF in urine stored at 4°C for zero, one, two, three, four, and five days following the addition of CTGF to urine samples. As shown in Figure 6, only N-terminal fragments of CTGF were detected in CTGF-supplemented urine after three days of storage at 4°C. After three days, CTGF was virtually undetectable in urine under these storage conditions. Therefore, detection and quantitation of N-terminal fragments of CTGF provided improved and more reliable detection and quantitation of the original amount of CTGF that was added to the urine. These results indicated that CTGF was less stable than N-terminal fragments in urine.

### Example 12: CTGF proteolysis in urine

The lower stability of CTGF (compared to N-terminal fragments of CTGF) in urine, or lack of the ability to detect and quantitate CTGF in urine, may have been due to instability and proteolysis of CTGF in human urine. To examine this, experiments similar to those described above in Example 11 were performed on human urine samples to which rhCTGF and various protease inhibitors were added. In this study, 10 ng/ml rhCTGF was added to normal human urine, which was then stored at 4°C for one to seven days in the presence or absence of protease inhibitors. For these experiments, CTGF detection and quantitation was performed using the CTGF ELISA assays as described above. The protease inhibitors used were a protease inhibitor cocktail (Sigma Chemical Co., catalog no. P-8340), N-hydroxy-2[[N'-(4-methoxy-benzenesulfonyl)-N'(4-chlorobenzyl)]amino]-acetamide (a metalloprotease inhibitor), or ethylenediamine tetra-acetic acid (EDTA). Results of CTGF proteolysis in urine are shown in Table 5 below.

**Table 5: CTGF Proteolysis in Urine**

| | Control Urine | Protease Inhibitor Mixture | Metallo-proteinase inhibitor | EDTA |
|---|---|---|---|---|
| N-C Assay (CTGF) | | | | |
| 1 day | 2.2 ng/ml | 3.5 ng/ml | 4.6 ng/ml | 3.5 ng/ml |
| 7 days | 0.0 ng/ml | 0.0 ng/ml | 0.5 ng/ml | 0.0 ng/ml |
| N-N Assay (N-terminal and CTGF) | | | | |
| 1 day | 10 ng/ml | 12 ng/ml | 14 ng/ml | 12 ng/ml |
| 7 days | 8 ng/ml | 14 ng/ml | 13 ng/ml | 7 ng/ml |

Table 5 above shows that after one day or seven days of storage, the majority of CTGF added to urine was undetectable in the absence of any added protease inhibitors. The inclusion of protease inhibitors resulted in higher levels of detectable CTGF in urine after one day of storage, compared to that in the absence of protease inhibitors. A similar extent of reduction in the loss of detectable CTGF occurred with a mixture of protease inhibitors, a metallo-proteinase inhibitor, as well as with EDTA, a metal ion chelator. These data indicated that proteases are responsible, at least in part, for the degradation, elimination, or loss of detectable CTGF in normal human urine during storage at 4°C. These results also suggested that detectable levels of CTGF were maintained in samples by the addition of protease inhibitors.

In contrast, N-terminal fragments of CTGF were readily detected and quantitated in these samples, either in the presence or absence of added protease inhibitors. Additionally, levels of N-terminal fragments of CTGF were consistent with the amount of rhCTGF exogenously added to the urine (10 ng/ml). Table 5 shows data determined using the N-N assay, which detects both CTGF and N-terminal fragments of CTGF. The levels of N-terminal fragments of CTGF were higher than the exogenously added 10 ng/ml rhCTGF due to endogenous levels of CTGF (i.e., N-terminal fragments of CTGF) in normal urine, which are approximately 1 to 2 ng/ml. In normal urine, CTGF and C-terminal fragments of CTGF were not detectable, whereas 1 to 2 ng/ml of N-terminal fragments of CTGF were detectable (data not shown). These results indicated that while CTGF was sensitive to protease activity, adding, at least in part, to the inability to detect and quantitate CTGF in urine, N-terminal fragments of CTGF appeared to be resistant to protease degradation, as measured by the ability to detect and quantitate N-terminal fragments of CTGF in urine.

### Example 13: CTGF in peritoneal dialysis patients

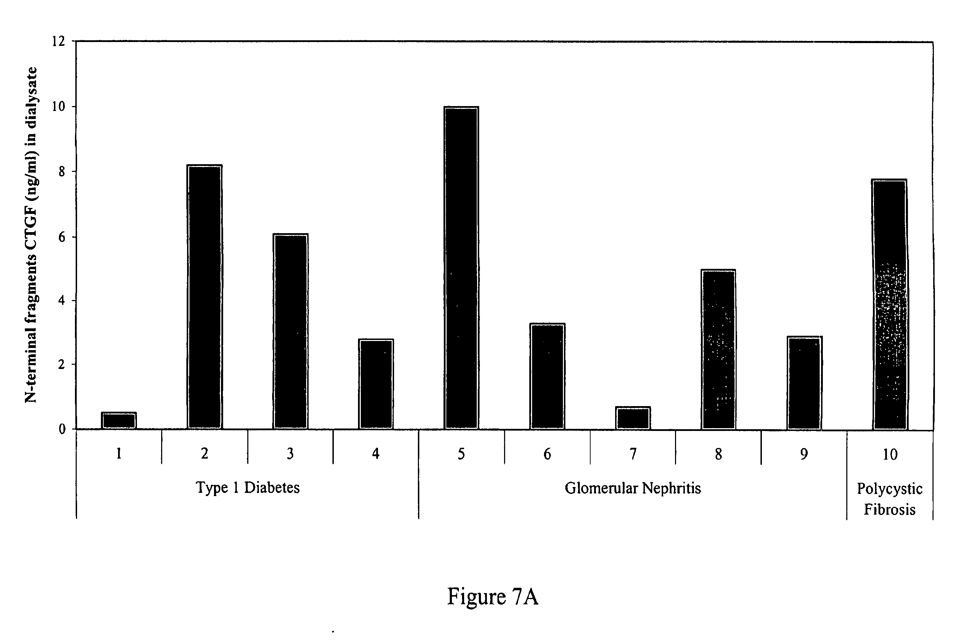

The levels of CTGF and fragments of CTGF in peritoneal dialysis patients were measured in dialysis fluids recovered from the peritoneal cavity of patients undergoing peritoneal dialysis for defective kidney function. Dialysis fluids were obtained and removed from the peritoneum of patients who had been undergoing peritoneal dialysis for 2 months or less (sample numbers 1, 2, 3, 6, and 7 in Figure 7A), or from patients who had been undergoing peritoneal dialysis for 50 to 100 months (sample numbers 4, 5, 8, 9, and 10 in Figure 7A). Five patients were diagnosed with type 1 diabetes, four patients had glomerular nephritis, and one patient was on dialysis as a result of polycystic kidney disease. In some cases, the peritoneal dialysis fluid was obtained from patients who had developed transient peritonitis while undergoing peritoneal dialysis. Sample numbers 4, 8, and 10 were from patients who had experienced bacterial peritonitis once previously, while sample number 9 was a patient who had experienced bacterial peritonitis four times previously.

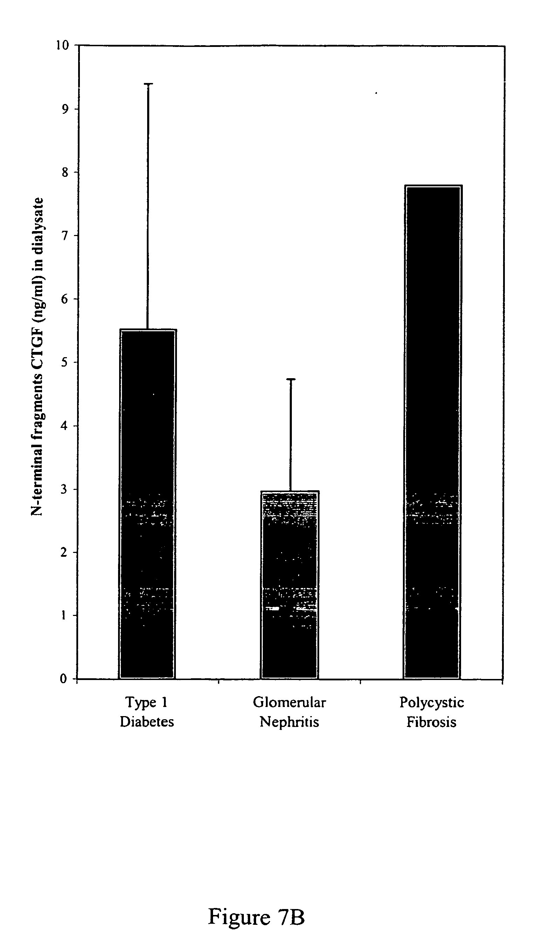

Detectable levels of N-terminal fragments of CTGF were found in all dialysis fluids examined (Figures 7A and 7B). The detection, quantitation, and comparison of CTGF, N-terminal fragments of CTGF, and C-terminal fragments of CTGF in the dialysate of peritoneal dialysis patients revealed highest levels of N-terminal fragments of CTGF (data not shown for CTGF and C-terminal fragments of CTGF).

### Example 14: CTGF in serum of renal fibrosis subjects

The presence of CTGF in the urine of kidney dialysis patients has been previously examined (Riser et al. (2000) J Am Soc Nephrol 11:25-38; International Patent Application WO 00/13706). To detect CTGF, these previous studies employed either heparin-sepharose to adsorb CTGF (thus resulting in the absence of N-terminal fragment detection due to N-terminal fragments of CTGF not adsorbing to heparin-sepharose beads), used an ELISA to CTGF that would not distinguish whole, N-terminal fragments, or C-terminal fragments of CTGF, and may not have possessed equal sensitivity of detection of each of the CTGF species (i.e., CTGF, N-terminal fragments of CTGF, and C-terminal fragments of CTGF).

In the present study, the levels of CTGF and fragments of CTGF in the serum of renal fibrosis patients were determined. Serum from normal donor patients and from renal fibrosis patients was obtained from Intergen Company (Purchase, NY). Patients had abnormal kidney functions and renal fibrosis resulting from the clinical manifestation of kidney transplant, chemical toxicity, or IgA-associated nephropathy.

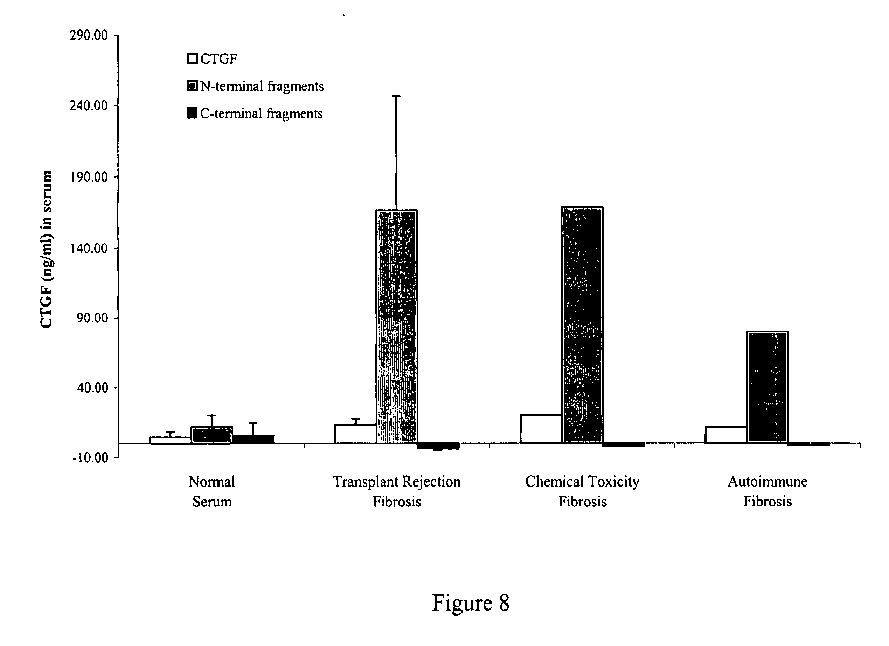

As shown in Figure 8, the levels of CTGF and C-terminal fragments of CTGF in the serum of renal fibrosis patients were essentially the same as or slightly lower than those found in the serum of normal individuals. These values, corresponding to the levels of CTGF or C-terminal fragments of CTGF in the serum of normal individuals or in renal fibrosis patients, were approximately 0-20 ng/ml. N-terminal fragments of CTGF, however, were readily detected in the serum of renal fibrosis patients. Additionally, the levels of N-terminal fragments of CTGF were elevated above that of CTGF and C-terminal fragments of CTGF in the serum of these patients. Therefore, the detection, quantitation, and comparison of CTGF, N-terminal fragments of CTGF, and C-terminal fragments of CTGF in the serum of these patients revealed significant elevations of N-terminal fragments of CTGF in patients diagnosed with renal fibrosis.

### Example 15: CTGF in serum of organ transplant subjects

The levels of CTGF and fragments of CTGF in the serum of organ transplantation patients were examined. Serum from patients with functional organ transplants (either liver or kidney) as well as serum from patients with chronic organ transplant rejection was obtained from Intergen Company. Patients with functional organ transplants were distinguished from those undergoing chronic rejection based on clinical examination of the patient plus measurements of creatinine clearance. Chronic organ transplant rejection was assessed greater than one year post-transplantation.

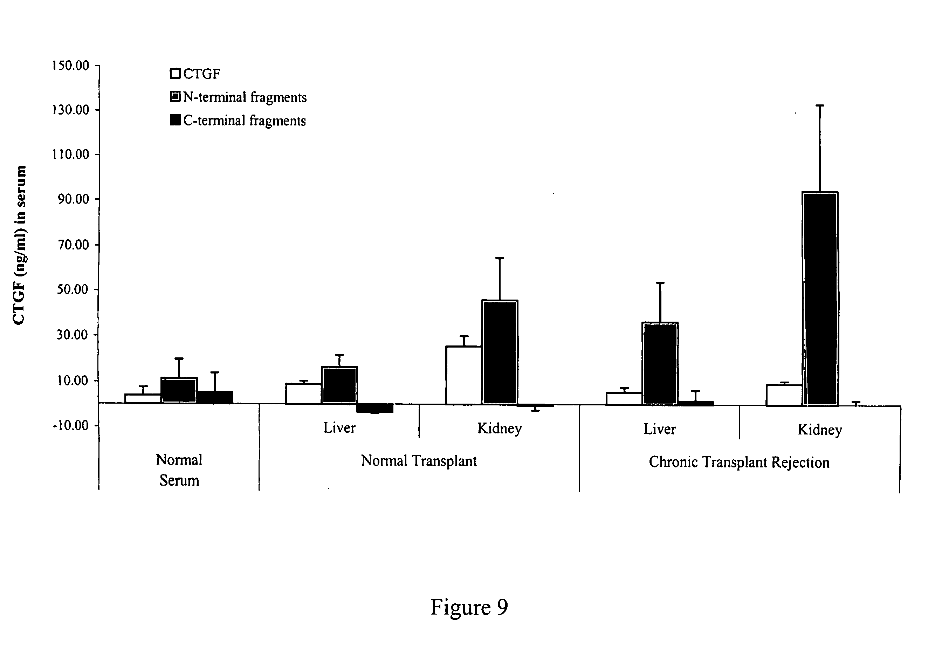

As shown in Figure 9, the levels of CTGF and C-terminal fragments of CTGF in the serum of patients with normal organ transplants or patients with chronic rejection following organ transplantation were essentially the same as that found in the serum of normal individuals (i.e., individuals who have not undergone organ transplantation). The values determined, corresponding to the levels of CTGF and C-terminal fragments of CTGF in the serum of normal individuals or in organ transplantation patients, were approximately 0 to 20 ng/ml for both CTGF and C-terminal fragments of CTGF.

In contrast, N-terminal fragments of CTGF were readily detected in the serum of organ transplant patients, as shown in Figure 9. Additionally, the levels of N-terminal fragments of CTGF were greatly elevated in the serum of patients with both normal organ transplantation and chronic transplant rejection, compared to that detected in the serum of healthy non-transplant individuals. Elevated levels of N-terminal fragments of CTGF were detected in the serum of individuals with liver transplants or kidney transplants. These results indicated that detection, quantitation, and comparison of the levels of CTGF, N-terminal fragments of CTGF, and C-terminal fragments of CTGF in the serum of organ transplant patients revealed the presence of elevated levels of N-terminal fragments of CTGF in patients having undergone organ transplant compared to non-transplant individuals. Additionally, the results showed that levels of N-terminal fragments of CTGF were further elevated in association with chronic rejection of organ transplants as compared to levels observed in normal organ transplants.

### Example 16: CTGF in serum of myocardial infarct subjects

The levels of CTGF and fragments of CTGF in the serum of myocardial infarct patients were examined. Serum from normal donor individuals and from myocardial infarct patients was obtained from Intergen Company. Myocardial infarction in patients was diagnosed symptomatically and further confirmed by assessment of troponin I levels in patient serum. In the ten patients examined, troponin I levels ranged from 14.1 to 103 ng/ml. The troponin I test method used was the AXSYM immunoassay system (Abbott Laboratories, Abbott Park IL), with a corresponding reference range of 0.0 to 0.5 ng/ml.

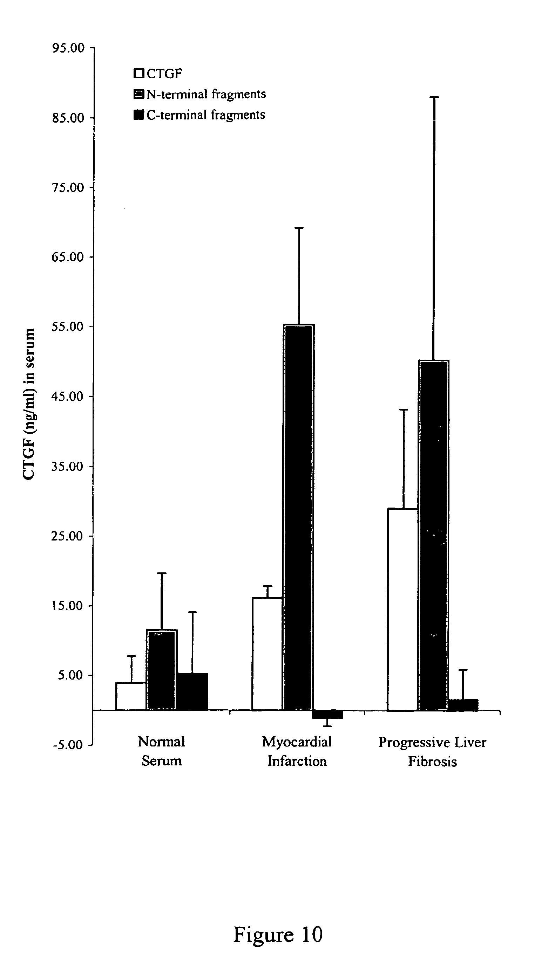

As shown in Figure 10, the levels of CTGF in the serum of myocardial infarct patients were only slightly above those determined in the serum of normal individuals. The levels of C-terminal fragments of CTGF in the serum of myocardial infarct subjects were essentially the same as or slightly lower than those found in the serum of normal individuals. N-terminal fragments of CTGF, however, were readily detected in the serum of myocardial infarct patients. Additionally, the levels of N-terminal fragments of CTGF were elevated above that of CTGF and C-terminal fragments of CTGF in the serum of these patients. Therefore, the detection, quantitation, and comparison of the levels of CTGF, N-terminal fragments of CTGF, and C-terminal fragments of CTGF in the serum of these patients revealed significant elevations of N-terminal fragments of CTGF in patients with evidence of a recent myocardial infarction.

### Example 17: CTGF in serum of liver fibrosis subjects

The levels of CTGF and fragments of CTGF in the serum of patients diagnosed with progressive liver fibrosis were examined. Serum from normal donor patients and from liver fibrosis patients was obtained from Intergen Company. As shown in Figure 10, low levels of CTGF, N-terminal fragments of CTGF, and C-terminal fragments of CTGF were detected in the serum obtained from normal individuals. These results indicated that in normal serum, the levels of CTGF, CTGF N-terminal fragments, and CTGF C-terminal fragments are in the range of approximately 0 to 20 ng/ml. The levels of C-terminal fragments of CTGF in the serum of patients with liver fibrosis were essentially the same as those found in the serum of normal individuals.

However, in the serum of patients with progressive liver fibrosis, the levels of CTGF and N-terminal fragments of CTGF were elevated compared to those in serum from normal individuals. Detection, quantitation, and comparison of the levels of CTGF, N-terminal fragments of CTGF, and C-terminal fragments of CTGF in the serum of these patients revealed the presence of elevated levels of N-terminal fragments of CTGF in patients with liver fibrosis.

### Example 18: CTGF in arthritic synovial fluid

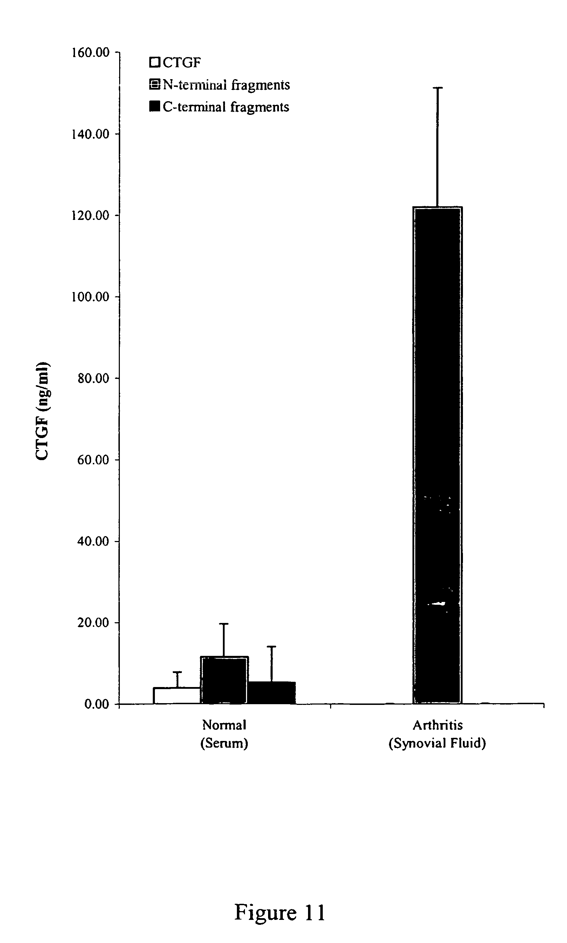

The levels of CTGF and fragments of CTGF in arthritic synovial fluids were determined. Serum from normal donor individuals was obtained from Intergen Company. Samples of synovial fluids from patients with inflammatory joint diseases, taken from patient knees, were obtained from Vital Products (Delray Beach FL). Levels of CTGF and C-terminal fragments of CTGF in arthritic synovial fluid were not detectable, as shown in Figure 11. In contrast, N-terminal fragments of CTGF were detected, and the levels of N-terminal fragments were elevated (compared to CTGF and C-terminal fragments of CTGF) in arthritic synovial fluid. Detection, quantitation, and comparison of the levels of CTGF, N-terminal fragments of CTGF, and C-terminal fragments of CTGF in the synovial fluid of these patients revealed the presence of elevated levels of N-terminal fragments of CTGF in patients diagnosed with inflammatory joint disease.

### Example 19: CTGF in vitreous fluid

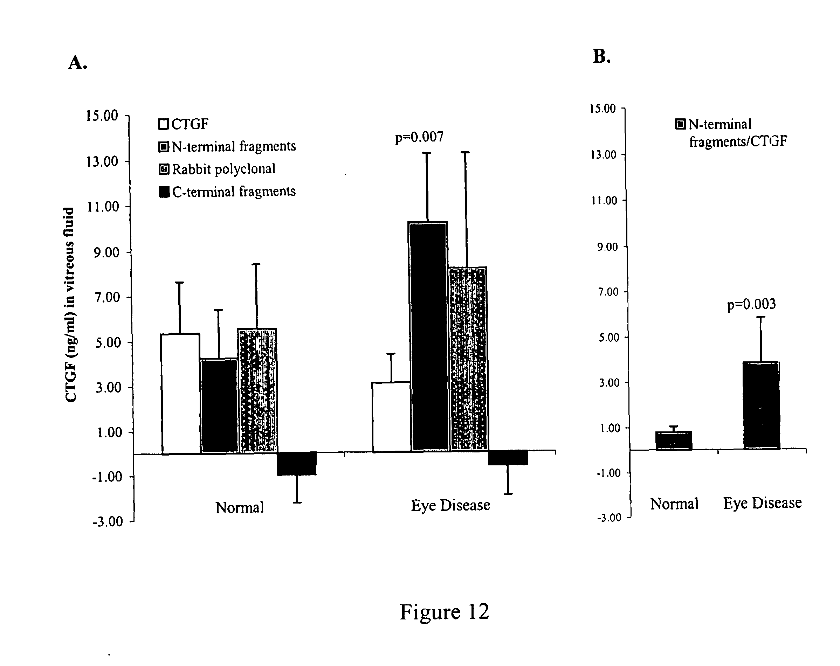

The levels of CTGF and fragments of CTGF in vitreous fluid from post-mortem patients diagnosed with eye disease were examined. Vitreous fluid was purchased from the Lions Eye Bank of Oregon (Portland OR). Figure 12A shows the results of CTGF assays performed on vitreous fluid drawn from subjects with and without clinically diagnosed eye disease. Eye diseases included type 1 diabetic retinopathy-induced blindness, type 2 diabetes with cataracts and mild macular degeneration, and non-diabetic bilateral cataracts. As shown in Figure 12A, CTGF N-terminal fragment levels were elevated in vitreous fluid of patients with clinically diagnosed eye disease compared to those in the vitreous fluid of patients without clinically diagnosed eye disease.

Data obtained for the levels of CTGF fragment expression can be presented as a ratio of the level of N-terminal fragments of CTGF in a sample to that of CTGF in the same sample, or as a ratio of the level of C-terminal fragments of CTGF in a sample to that of CTGF in the same sample. Mathematical transformation was performed using the data obtained for the levels of CTGF and N-terminal fragments of CTGF in vitreous fluids (Figure 12A). The ratio of the levels of N-terminal fragments of CTGF to CTGF was determined, using the data described above (samples from vitreous fluids), the results of which are shown in Figure 12B (N-terminal fragment/CTGF). The ratio of the levels of N-terminal fragments of CTGF to that of CTGF showed further evidence that determining the levels of N-terminal fragments of CTGF provided a more sensitive and reliable assessment of CTGF expression in biological samples.

The data were analyzed further to determine statistical significance of the values obtained for the levels of CTGF and fragments thereof between samples from normal versus diseased subjects. A T-test analysis was performed on the data shown in Figure 12. Using the CTGF assay (N-C assay), the difference in the levels of CTGF obtained in samples from normal compared to disease vitreous fluid was not statistically significant, as determined by T-test (P = 0.150).

Additionally, an ELISA assay for CTGF using a rabbit polyclonal antibody to CTGF was also performed. This antibody preparation was produced in rabbits immunized with CTGF (using standard procedures known in the art), and therefore contains multiple antibodies recognizing multiple epitopes within the CTGF polypeptide. The ELISA assay to detect and quantitate CTGF using this rabbit polyclonal antibody was performed using procedures known in the art. The rabbit polyclonal antibody assay to CTGF cannot distinguish CTGF, N-terminal fragments of CTGF, or C-terminal fragments of CTGF. Results of the ELISA assay using a rabbit polyclonal antibody are shown in Figure 12A (Rabbit Polyclonal). Analysis of the data obtained for CTGF levels using the rabbit polyclonal antibody to CTGF indicated that the levels of CTGF in samples from normal compared to disease vitreous fluid were not statistically significant, as determined by T-test (P = 0.317). This indicated that the present methods that distinguish CTGF, N-terminal fragments of CTGF, or C-terminal fragments of CTGF, did reliably compare CTGF levels in normal versus diseased states.

In contrast, T-test analysis of the values obtained with an assay measuring the levels of CTGF N-terminal fragments showed a statistically significant difference in the amount of N-terminal fragments of CTGF in vitreous fluid in samples from normal compared to diseased individuals (*P* = 0.007). A T-test performed on the ratio of the levels of N-terminal fragments of CTGF to that of CTGF also indicated a significant difference in the amount of N-terminal fragments of CTGF in vitreous fluid in samples from normal compared to diseased individuals (*P* = 0.003). Therefore, detecting and measuring the levels of CTGF alone did not provide statistically significant data distinguishing the levels of CTGF in normal versus diseased conditions. Detecting and measuring the levels of N-terminal fragments of CTGF, as described in the present invention, however, did provide statistically significant data distinguishing the levels of N-terminal fragments of CTGF in normal versus diseased conditions.

### Example 20: CTGF in serum of cancer patients

The levels of CTGF and fragments of CTGF in the serum of cancer patients were determined. Serum from normal donor patients and from cancer patients was obtained from Intergen Company. Detection and quantitation of the following cancer markers were performed by Intergen Company.

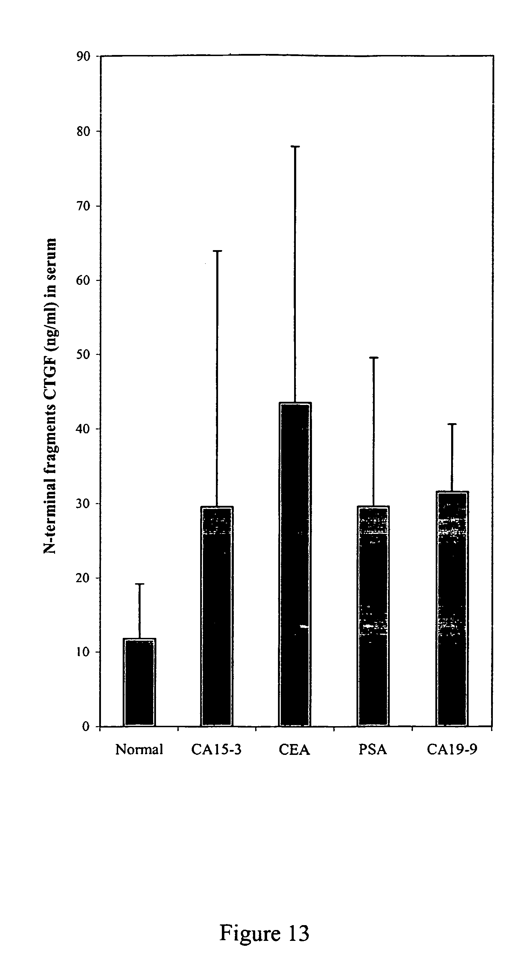

CA 15-3 is a mucin-like membrane glycoprotein that is shed from tumor cells into the bloodstream. CA 15-3 is a serum cancer antigen that has been used in the management of patients with breast cancer. CA 15-3 levels were determined using a heterogenous sandwich magnetic separation kit and reagents from Bayer Diagnostics (Tarrytown NY). Patients were considered positive for CA 15-3 if values greater than 30 U/ml were obtained in their serum. Assay standard reference range was 0 to 30 U/ml. All CA 15-3 patients were female. Ages ranged from 45 to 88 years old, and CA 15-3 levels ranged from 32 to 1,292 U/ml. These samples were defined as 'breast and ovarian' cancer samples (sample group CA15-3 in Figure 13). One third of all CA 15-3 positive patients tested had CA 15-3 levels greater than 10-fold above the standard clinical reference level of 30 U/ml.

Carcinoembryonic antigen (CEA) belongs to a family of cell-surface glycoproteins with increased expression found in a variety of malignancies. CEA is a cancer antigen used in monitoring gastrointestinal tract and breast cancers. CEA levels were determined using a heterogenous sandwich magnetic separation kit and reagents from Bayer Diagnostics. Patients were considered positive for CEA if values greater than 5 ng/ml were obtained in their serum. Assay standard reference range was from 0.1 to 4.9 ng/ml. CEA positive patients included both male and female, from 30 to 79 years of age, with CEA levels between 5.5 to 2,950 ng/ml. These patient samples were defined as 'colorectal, GI, and breast' cancer samples (sample group CEA in Figure 13). One third of all CEA positive patients tested had CEA levels greater than 10-fold above the standard clinical reference range.

Prostate specific antigen (PSA) is a glycoprotein produced primarily by epithelial cells that line the acini and ducts of the prostate gland. Elevated serum PSA levels have become an important marker of prostate pathologies, and are used as a tumor marker for early detection of prostate cancer. PSA levels were determined using a heterogenous sandwich magnetic separation kit and reagents from Bayer Diagnostics. Patients were considered positive for PSA if values greater than 8.0 ng/ml were obtained in their serum. Assay standard reference range was from 0 to 4 ng/ml. All PSA positive patients were male. Ages ranged from 55 to 96 years old, and PSA levels ranged from 8.3 to 1,919.0 ng/ml. These samples were defined as 'prostate' cancer samples (sample group PSA in Figure 13). One third of all PSA positive patients tested had PSA levels greater than 10-fold above the standard clinical reference range.

CA 19-9 is a tumor related mucin that contains the sialylated Lewis-A pentasaccharide epitope, lacto-N-fucopentaose II. CA 19-9 is produced by adenocarcinomas of the pancreas, stomach, gall bladder, colon, ovary, and lung, and is shed into the circulation. CA 19-9 levels were determined by Bayer Diagnostics. These samples were defined as 'pancreas, GI, and liver' cancer samples (sample group CA19-9 in Figure 13).

As shown in Figure 13, CTGF N-terminal fragment levels were elevated in the serum of cancer patients with various types of cancers as determined by elevated levels of certain cancer markers (as described above and shown in Figure 13), as compared to CTGF N-terminal fragment levels in the serum of normal individuals. Detection, quantitation, and comparison of the levels of CTGF, N-terminal fragments of CTGF, and C-terminal fragments of CTGF in the serum of these patients revealed the presence of elevated levels of N-terminal fragments of CTGF in patients diagnosed with various forms of cancer (data not shown for the levels of CTGF and C-terminal fragments of CTGF).

### Example 21: N-terminal fragments of CTGF in urine of Type I diabetes subjects

The levels of N-terminal fragments of CTGF in the urine of subjects diagnosed with type I diabetes were detected and quantitated using the CTGF ELISA assay described above. The extent of kidney tubular damage in patients with type I diabetes was assessed by measuring the amount of albumin in their urine (albuminuria), an often-used indicator of advanced kidney disease such as diabetic nephropathy. Patients were considered to have a positive microalbuminuria test result with urine protein (albumin) excretion levels of 20 to 200 µg/minute. Macroalbuminuria was defined as urine protein (albumin) excretion levels in excess of 200 µg/minute. Patients were considered to be normal for albuminuria with urine protein (albumin) excretion levels of less than 20 µg/minute. Type I diabetic patients were therefore categorized as normal, microalbuminuria, or macroalbuminuria, based on the results of the albuminuria tests performed.

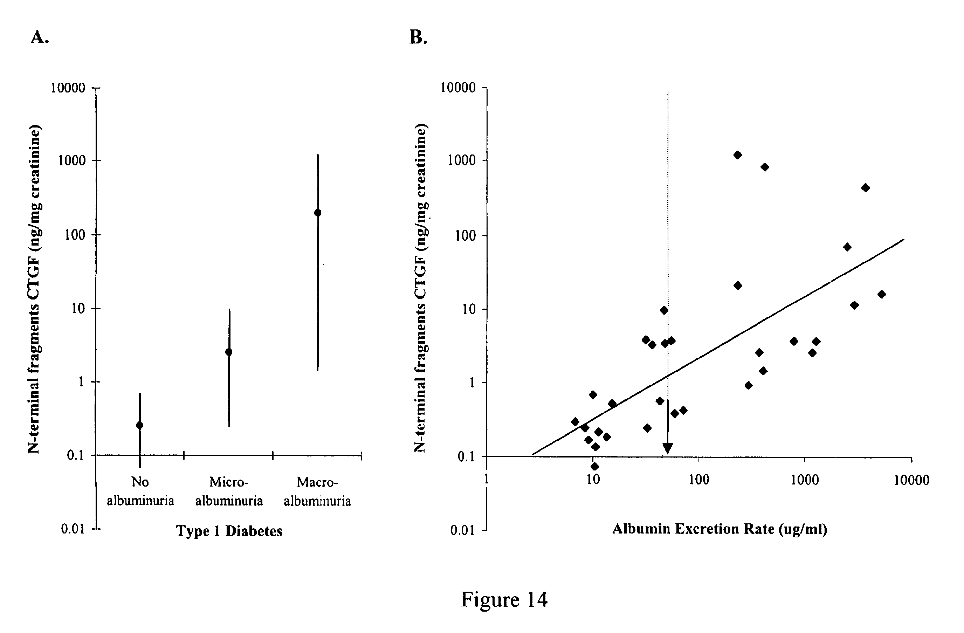

The levels of N-terminal fragments of CTGF were measured in the urine of patients with type I diabetes, and compared to the measured levels of urine creatinine. The data obtained for the levels of N-terminal fragments of CTGF in these individuals was converted (mathematical transformation) to logarithmic scale, as shown in Figure 14A. The results are presented as nanograms of N-terminal fragments of CTGF per milligram of creatinine. As shown in Figure 14A, elevated levels of N-terminal fragments of CTGF were detected in the urine from type I diabetes subjects with microalbuminuria and macroalbuminuria, as compared to that of type I diabetes patients with no albuminuria. CTGF or C-terminal fragments of CTGF were not detected in these samples (data not shown). The levels of N-terminal fragments of CTGF (nanograms) per milligram creatinine were highest in the urine of patients characterized as having macroalbuminuria.

Graphing the results on logarithmic scale following mathematical transformation of the data revealed that the levels of N-terminal CTGF fragments measured in urine correlated with the extent of kidney tubular damage (as measured by albuminuria/creatinine levels) in the groups measured (i.e., patients with normal albuminuria, microalbuminuria, or macroalbuminuria). (See Figure 14A)

The levels of N-terminal fragments of CTGF measured in the urine of type I diabetes patients were also compared to the rate of albumin excretion into the urine of these individuals. Measurement of the rate of albumin excretion (presented as micrograms albumin per minute) into urine is often used to indicate the extent of kidney damage or severity of kidney disease. As kidney damage increases, and proper or normal functions of the kidney are further impaired, a higher rate of albumin excretion into the urine is observed, compared to that observed in normal kidney. As show in Figure 14B, increased levels of N-terminal fragments of CTGF (presented as nanograms of N-terminal fragments of CTGF per milligram creatinine) correlated well with increased rates of albumin excretion in urine from type I diabetes patients. The arrow in Figure 14B indicates the uppermost limit of albumin excretion rate in normal individuals.

### Example 22: CTGF in plasma of scleroderma patients

Scleroderma is a connective tissue disease characterized by fibrotic, degenerative, and inflammatory changes in the skin, blood vessels, skeletal muscles, and internal organs. Damage to the cells lining the walls of small arteries and an abnormal buildup of tough scar-like fibrous tissue in the skin are hallmarks of the disease. Patients with scleroderma may develop either a localized or systemic form of the disease. In localized scleroderma, areas on the skin of the hands and face are most often affected. In systemic scleroderma, the organs of the body, widespread areas of the skin, or both, may be involved. Systemic scleroderma, also known as systemic sclerosis, has two primary variants, called limited and diffuse scleroderma, both forms of which are progressive. The effects of limited scleroderma can be widespread, but most often the internal organs are not affected. Diffuse scleroderma can affect wide areas of the skin, connective tissue, and other organs. Death of affected individuals may occur from gastrointestinal, cardiac, kidney, or pulmonary involvement.

The levels of CTGF and fragments of CTGF were measured in the plasma of individuals with diffuse or with limited scleroderma, using methods described above, and compared to that seen in the plasma of normal individuals. No significant differences in the levels of CTGF or C-terminal fragments of CTGF were observed between normal individuals and individuals with diffuse or limited scleroderma. However, a difference in the levels of N-terminal fragments of CTGF was seen in the plasma of individuals with either diffuse or limited scleroderma, compared to that observed in normal individuals. Therefore, the detection, quantitation, and comparison of CTGF, N-terminal fragments of CTGF, and C-terminal fragments of CTGF in the plasma of these individuals revealed elevations in the levels of N-terminal fragments of CTGF in individuals with diffuse or limited scleroderma, which was not observed by determining the levels of CTGF or of C-terminal fragments of CTGF.

### Example 23: CTGF in pulmonary fibrosis

Lung injury is a frequent side effect observed in anticancer therapy. Application of high doses of ionizing radiation is considered to cause deleterious pneumonitis and pulmonary fibrosis. Radiation pneumonitis, which ultimately leads to pulmonary fibrosis, is thought to be caused by radiation-induced local cytokine production confined to the field of irradiation. When radiation is combined with radio-sensitizing cytotoxic drugs, the ensuing fibrosis appears to be more pronounced and more rapid in onset than that observed with radiation treatment alone. The specific pathophysiologic changes associated with the progression from pneumonitis to fibrosis have not been fully defined. Measuring and determining loss of lung function in these patients is currently employed to determine the extent and progression of fibrosis.

Experimental evidence indicates that CTGF expression is elevated following lung injury. For example, CTGF levels have been shown to be elevated in bleomycin-induced lung fibrosis in mice. (Lasky et al. (1998) Am J Physiol 275:L365-L371.) Additionally, CTGF mRNA levels were increased by more that 10-fold in bronchoalveolar lavage (BAL) fluid from patients with interstitial pulmonary fibrosis (IPF), by more than 40-fold in stage I/II sarcodoisis patients, and by more than 90-fold in stage III/IV sarcodoisis patients, compared to that present in BAL fluid from healthy, non-smoking control patients without lung fibrosis. (Allen et al. (1999) Am J Respir Cell Mol Biol 21:693-700.) Therefore, it would be expected that CTGF and fragments of CTGF would be elevated in plasma and BAL fluid from patients having undergone radiation exposure either with or without combination therapy involving radio-sensitizing cytotoxic drugs. Changes in the levels of CTGF or fragments of CTGF in plasma, sputum, or lung lavage would thus serve as a diagnostic measure of changes in lung pathology occurring in patients undergoing radiation therapy. Therefore, monitoring the levels of CTGF and fragments of CTGF following such therapeutic treatments, using methods of the present invention, would provide a diagnostic predictor of a fibrotic response which would occur following specific therapies.

### Example 24: CTGF in schistosomiasis

Advanced cases of schistosomiasis are characterized by the presence of schistosomal eggs in the liver. In untreated patients, a granulomatous response to the eggs is followed by the development of extensive hepatic fibrosis and hepatomegaly. Periportal fibrosis is accompanied by splenomegaly, portal vein dilatation, and the development of portosystemic collaterals. (Kardorff et al (1999) Acta Tropica 73:153-164.) These changes are positively correlated with an increase in serum levels of fibrosis markers, such as carboxyterminal procollagen IV peptide (NC1) and hyaluronan. (Kardorff et al. (1999) *supra,* and Ricard-Blum et al. (1999) Am J Trop Med Hyg 60:658-663.)

Changes in the levels of CTGF or fragments of CTGF in plasma, urine, or tissue biopsies would serve as a diagnostic measure of changes in liver pathology occurring in individuals affected with schistosomiasis. Therefore, monitoring the levels of CTGF and fragments of CTGF in such individuals, using methods of the present invention, would provide a diagnostic predictor of a fibrotic response in the liver following infection and progression of schistosomiasis.

### Example 25: CTGF in inflammatory and infectious disorders

Constrictive bronchiolitis, also described as obliterative bronchiolitis in lung transplant patients, involves inflammation and fibrosis occurring predominantly in the walls and contiguous tissues of membranous and respiratory bronchioles with resultant narrowing of their lumens. Constrictive bronchiolitis is most often a complication of lung and heart-lung transplantation, but is also associated with bone marrow transplantation. Constrictive bronchiolitis is also associated with rheumatoid arthritis, after inhalation of toxic agents, after ingestion of certain drugs, and as a rare complication of adenovirus, influenza type A, measles, and *Mycoplasma pneumoniae* infections in children. Currently, histopathogenic diagnosis of constrictive bronchiolitis in lung transplant and other patients has been difficult to make, due to the patchy distribution of lesions, the technical difficulty in obtaining tissue in late lesions with extensive fibrosis, and the failure to recognize lesions. In early stages of the disease, constrictive bronchiolitis may be subtle and easily missed in routine hematoxylin-eosin-stained specimens, while in advanced stages, the disease may be equally difficult to diagnose if the patchy scarring in the lung is interpreted as nonspecific.

Other examples of inflammatory and infectious diseases associated with a fibrotic response, wherein detecting and quantitating the levels of CTGF and fragments of CTGF would be useful in the diagnosis and prognosis of such diseases, include the following: primary biliary cirrhosis, an autoimmune disease that predominantly affects women and is characterized by chronic progressive destruction of small intrahepatic bile ducts with portal inflammation and ultimately fibrosis; idiopathic pulmonary fibrosis, recently characterized as having adenovirus involvement in the pathogenesis of idiopathic pulmonary fibrosis or interstitial pneumonia associated with collagen vascular disease; and renal parenchymal infection, such as pyelonephritis (i.e., inflammation of the parenchyma of the kidney and the lining of the renal pelvis of the kidney, often due to bacterial infection), believed to be a prerequisite for acquired (postnatal) renal scarring.

Changes in the levels of CTGF or fragments of CTGF in plasma, urine, or tissue biopsies would serve as a diagnostic measure of the initiation and progression of a fibrotic response in tissues occurring following infection and inflammation. Therefore, monitoring the levels of CTGF and fragments of CTGF in such individuals, using methods of the present invention, would provide a diagnostic predictor and a method of monitoring the progression of a fibrotic response following infection inflammation associated with a variety of disorders.

Various modifications of the invention, in addition to those shown and described herein, will become apparent to those skilled in the art from the foregoing description.

## Claims

1. A method for diagnosing a CTGF-associated disorder or for prognosis of a CTGF-associated disorder, the method comprising:
(a) contacting a sample from a subject with a first antibody that specifically binds to a CTGF N-terminal fragment region from residue 1 to residue 198 of SEQ ID NO:2, under conditions suitable for binding;
(b) optionally isolating the bound first antibody of step (a);
(c) adding a second antibody to the sample of step (a) or the isolate of step (b) that specifically binds to a CTGF N-terminal fragment region from residue 1 to residue 198 of SEQ ID NO:2, that is different from the region bound by the first antibody, under conditions suitable for binding;
(d) removing unbound second antibody of step (c); and
(e) quantitating the amount of bound second antibody, wherein the amount of bound second antibody corresponds to the level of polypeptide comprising CTGF N-terminal fragment in the sample; and
(f) comparing the level of polypeptide comprising CTGF N-terminal fragment in the sample with a standard level, wherein an increased or decreased amount of polypeptide comprising CTGF N-terminal fragment in the sample is indicative of the presence of a CTGF-associated disorder.

2. A method for monitoring the progression of a CTGF-associated disorder in a subject, the method comprising:
(a) contacting a first sample obtained from the subject at a first point in time, and a second sample obtained from the subject at a second point in time, with a first antibody that specifically binds to a CTGF N-terminal fragment region from residue 1 to residue 198 of SEQ ID NO:2 under conditions suitable for binding;
(b) optionally isolating the bound first antibody of step (a) from the first and second samples;
(c) adding a second antibody to the samples of step (a) or the isolates of step (b) that specifically binds to a CTGF N-terminal fragment region from residue 1 to residue 198 that is different from the region bound by the first antibody, under conditions suitable for binding;
(d) removing unbound second antibody of step (c);
(e) quantitating the amount of bound second antibody, wherein the amount of bound second antibody in the isolates corresponds to the level of polypeptide comprising CTGF N-terminal fragment in the first and second sample; and
(f) comparing the level of polypeptide comprising CTGF N-terminal fragment in the first sample to the level of polypeptide comprising CTGF N-terminal fragment in the second sample, wherein a difference between the level of polypeptide comprising CTGF N-terminal fragment in the first sample and the level of polypeptide comprising CTGF N-terminal fragment in the second sample is indicative of the progression of a CTGF-associated disorder.

3. The method of claim 1 or claim 2, wherein the first antibody is bound to a substrate.

4. The method of any preceding claim, wherein the first antibody is a functional antibody fragment.

5. The method of any preceding claim, wherein the second antibody is attached to a detectable label.

6. The method of claim 5, wherein the detectable label is selected from the group consisting of fluorophores, radioactive isotopes, metals, and enzyme conjugates.

7. The method of any preceding claim, wherein the second antibody is a functional antibody fragment.

8. The method of any preceding claim, wherein the sample is obtained from a mammal.

9. The method of claim 8, wherein the mammal is a human.

10. The method of any preceding claim, wherein the sample is selected from urine or plasma.

11. The method of any preceding claim, wherein the CTGF-associated disorder is selected from the group consisting of renal fibrosis, liver fibrosis, cardiac fibrosis, inflammatory joint disease, cancer, diabetes, scleroderma, organ transplant, peritoneal dialysis, or myocardial infarction.

12. A kit for diagnosing a CTGF-associated disorder or for prognosis of a CTGF-associated disorder, the kit comprising:
(a) a first antibody which binds specifically to an N-terminal fragment region on CTGF from residue 1 to residue 198 of SEQ ID NO:2; and
(b) a second antibody which binds specifically to an N-terminal fragment region on CTGF from residue 1 to residue 198 of SEQ ID NO:2 different from the region bound by the first antibody.

13. A method according to claim 1 or claim 2, or a kit according to claim 12, wherein the N-terminal fragment region on CTGF is from residue 23 to residue 179 of SEQ ID NO:2.

## Patentansprüche

1. Verfahren zur Diagnose einer mit CTGF in Zusammenhang stehenden Erkrankung oder für die Prognose einer mit CTGF in Zusammenhang stehenden Erkrankung, bei dem man:
(a) eine Probe aus einem Patienten mit einem ersten Antikörper, der spezifisch an einen N-terminalen CTGF-Fragmentbereich von Rest 1 bis Rest 198 der SEQ ID NO:2 bindet, unter für die Bindung geeigneten Bedingungen in Kontakt bringt;
(b) gegebenenfalls den gebundenen ersten Antikörper aus Schritt (a) isoliert;
(c) die Probe aus Schritt (a) oder das Isolat aus Schritt (b) mit einem zweiten Antikörper, der spezifisch an einen N-terminalen CTGF-Fragmentbereich von Rest 1 bis Rest 198 der SEQ ID NO:2, welcher sich von dem durch den ersten Antikörper gebundenen Bereich unterscheidet, bindet, unter für die Bindung geeigneten Bedingungen versetzt;
(d) nicht gebundenen zweiten Antikörper aus Schritt (c) abtrennt; und
(e) die Menge an gebundenem zweiten Antikörper bestimmt, wobei die Menge an gebundenem zweiten Antikörper der Konzentration an N-terminales CTGF-Fragment umfassendem Polypeptid in der Probe entspricht; und
(f) die Konzentration an N-terminales CTGF-Fragment umfassendem Polypeptid in der Probe mit einer Standardkonzentration vergleicht, wobei eine erhöhte oder reduzierte Menge an N-terminales CTGF-Fragment umfassendem Polypeptid in der Probe das Vorliegen einer mit CTGF in Zusammenhang stehenden Erkrankung anzeigt.

2. Verfahren zur Überwachung des Fortschreitens einer mit CTGF in Zusammenhang stehenden Erkrankung bei einem Patienten, wobei man bei dem Verfahren:
(a) eine dem Patienten zu einem ersten Zeitpunkt entnommene erste Probe und eine dem Patienten zu einem zweiten Zeitpunkt entnommene zweite Probe mit einem ersten Antikörper, der spezifisch an einen N-terminalen CTGF-Fragmentbereich von Rest 1 bis Rest 198 der SEQ ID NO:2 bindet, unter für die Bindung geeigneten Bedingungen in Kontakt bringt;
(b) gegebenenfalls den gebundenen ersten Antikörper aus Schritt (a) aus der ersten und der zweiten Probe isoliert;
(c) die Proben aus Schritt (a) oder die Isolate aus Schritt (b) mit einem zweiten Antikörper, der spezifisch an einen N-terminalen CTGF-Fragmentbereich von Rest 1 bis Rest 198, welcher sich von dem durch den ersten Antikörper gebundenen Bereich unterscheidet, bindet, unter für die Bindung geeigneten Bedingungen versetzt;
(d) nicht gebundenen zweiten Antikörper aus Schritt (c) abtrennt;
(e) die Menge an gebundenem zweiten Antikörper bestimmt, wobei die Menge an gebundenem zweiten Antikörper in den Isolaten der Konzentration an N-terminales CTGF-Fragment umfassendem Polypeptid in der ersten und zweiten Probe entspricht; und
(f) die Konzentration an N-terminales CTGF-Fragment umfassendem Polypeptid in der ersten Probe mit der Konzentration an N-terminales CTGF-Fragment umfassendem Polypeptid in der zweiten Probe vergleicht, wobei ein Unterschied zwischen der Konzentration an N-terminales CTGF-Fragment umfassendem Polypeptid in der ersten Probe und der Konzentration an N-terminales CTGF-Fragment umfassendem Polypeptid in der zweiten Probe das Fortschreiten einer mit CTGF in Zusammenhang stehenden Erkrankung anzeigt.

3. Verfahren nach Anspruch 1 oder Anspruch 2, wobei der erste Antikörper an ein Substrat gebunden ist.

4. Verfahren nach einem vorhergehenden Anspruch, wobei es sich bei dem ersten Antikörper um ein funktionelles Antikörperfragment handelt.

5. Verfahren nach einem vorhergehenden Anspruch, wobei der zweite Antikörper an eine nachweisbare Markierung gebunden ist.

6. Verfahren nach Anspruch 5, wobei die nachweisbare Markierung aus der aus Fluorophoren, radioaktiven Isotopen, Metallen und Enzymkonjugaten bestehenden Gruppe ausgewählt ist.

7. Verfahren nach einem vorhergehenden Anspruch, wobei es sich bei dem zweiten Antikörper um ein funktionelles Antikörperfragment handelt.

8. Verfahren nach einem vorhergehenden Anspruch, wobei die Probe einem Säuger entnommen wird.

9. Verfahren nach Anspruch 8, wobei es sich bei dem Säuger um einen Menschen handelt.

10. Verfahren nach einem vorhergehenden Anspruch, wobei die Probe aus Urin oder Plasma ausgewählt ist.

11. Verfahren nach einem vorhergehenden Anspruch, wobei die mit CTGF in Zusammenhang stehende Erkrankung aus der aus Nierenfibrose, Leberfibrose, Herzfibrose, entzündlicher Gelenkerkrankung, Krebs, Diabetes, Skleroderm, Organtransplantation, peritonealer Dialyse oder Myokardinfarkt bestehenden Gruppe ausgewählt ist.

12. Kit zur Diagnose einer mit CTGF in Zusammenhang stehenden Erkrankung oder für die Prognose einer mit CTGF in Zusammenhang stehenden Erkrankung, wobei das Kit Folgendes umfasst:
(a) einen ersten Antikörper, der an einen N-terminalen Fragmentbereich auf CTGF von Rest 1 bis Rest 198 der SEQ ID NO:2 spezifisch bindet; und
(b) einen zweiten Antikörper, der an einen von dem durch den ersten Antikörper gebundenen Bereich verschiedenen N-terminalen Fragmentbereich auf CTGF von Rest 1 bis Rest 198 der SEQ ID NO:2 spezifisch bindet.

13. Verfahren gemäß Anspruch 1 oder Anspruch 2 oder Kit gemäß Anspruch 12, wobei sich der N-terminale Fragmentbereich auf CTGF von Rest 23 bis Rest 179 der SEQ ID NO:2 erstreckt.

## Revendications

1. Procédé pour le diagnostic d'un trouble associé au CTGF ou pour le pronostic d'un trouble associé au CTGF, le procédé comprenant :
(a) la mise en contact d'un échantillon provenant d'un sujet avec un premier anticorps qui se lie spécifiquement à une région fragment N-terminal du CTGF, du résidu 1 au résidu 198 de SEQ ID NO:2, dans des conditions convenant à une liaison ;
(b) en option, l'isolement du premier anticorps lié de l'étape (a) ;
(c) l'addition d'un deuxième anticorps à l'échantillon de l'étape (a) ou de l'isolat de l'étape (b), qui se lie spécifiquement à une région fragment N-terminal du CTGF, du résidu 1 au résidu 198 de SEQ ID NO:2, qui est différente de la région à laquelle est lié le premier anticorps, dans des conditions convenant à une liaison ;
(d) l'enlèvement du deuxième anticorps non lié de l'étape (c) ; et
(e) la quantification de la quantité du deuxième anticorps lié, la quantité du deuxième anticorps lié correspondant au niveau de polypeptide comprenant le fragment N-terminal du CTGF dans l'échantillon ; et
(f) la comparaison du niveau du polypeptide comprenant le fragment N-terminal du CTGF dans l'échantillon à un niveau standard, une augmentation ou une diminution de la quantité du polypeptide comprenant le fragment N-terminal du CTGF dans l'échantillon étant une indication de la présence d'un trouble associé au CTGF.

2. Procédé pour la surveillance de la progression d'un trouble associé au CTGF chez un sujet, le procédé comprenant :
(a) la mise en contact d'un premier échantillon obtenu du sujet à un premier instant, et d'un deuxième échantillon obtenu du sujet à un deuxième instant, avec un premier anticorps qui se lie spécifiquement à une région fragment N-terminal du CTGF, du résidu 1 au résidu 198 de SEQ ID NO:2, dans des conditions convenant à une liaison ;
(b) en option, l'isolement, à partir du premier et du deuxième échantillon, du premier anticorps lié de l'étape (a) ;
(c) l'addition d'un deuxième anticorps aux échantillons de l'étape (a) ou aux isolats de l'étape (b), qui se lie spécifiquement à une région fragment N-terminal du CTGF, du résidu 1 au résidu 198, qui est différente de la région à laquelle est lié le premier anticorps, dans des conditions convenant à une liaison ;
(d) l'enlèvement du deuxième anticorps non lié de l'étape (c) ;
(e) la quantification de la quantité du deuxième anticorps lié, la quantité du deuxième anticorps lié dans les isolats correspondant au niveau du polypeptide comprenant le fragment N-terminal du CTGF dans le premier et le deuxième échantillon ; et
(f) la comparaison du niveau du polypeptide comprenant le fragment N-terminal du CTGF dans le premier échantillon au niveau du polypeptide comprenant le fragment N-terminal du CTGF dans le deuxième échantillon, une différence entre le niveau du polypeptide comprenant le fragment N-terminal du CTGF dans le premier échantillon et le niveau du polypeptide comprenant le fragment N-terminal du CTGF dans le deuxième échantillon étant une indication de la progression d'un trouble associé au CTGF.

3. Procédé de la revendication 1 ou de la revendication 2, dans lequel le premier anticorps est lié à un substrat.

4. Procédé de l'une quelconque des revendications précédentes, dans lequel le premier anticorps est un fragment d'anticorps fonctionnel.

5. Procédé de l'une quelconque des revendications précédentes, dans lequel le deuxième anticorps est fixé à un marqueur détectable.

6. Procédé de la revendication 5, dans lequel le marqueur détectable est choisi dans le groupe consistant en les fluorophores, les radioisotopes, les métaux et les conjugués enzymatiques.

7. Procédé de l'une quelconque des revendications précédentes, dans lequel le deuxième anticorps est un fragment d'anticorps fonctionnel.

8. Procédé de l'une quelconque des revendications précédentes, dans lequel l'échantillon est obtenu d'un mammifère.

9. Procédé de la revendication 8, dans lequel le mammifère est un humain.

10. Procédé de l'une quelconque des revendications précédentes, dans lequel l'échantillon est choisi parmi l'urine ou le plasma.

11. Procédé de l'une quelconque des revendications précédentes, dans lequel le trouble associé au CTGF est choisi dans le groupe consistant en la fibrose rénale, la fibrose hépatique, la fibrose cardiaque, la maladie inflammatoire des articulations, le cancer, le diabète, la sclérodermie, la transplantation d'organe, la dialyse péritonéale ou l'infarctus du myocarde.

12. Trousse pour le diagnostic d'un trouble associé au CTGF ou pour le pronostic d'un trouble associé au CTGF, la trousse comprenant :
(a) un premier anticorps qui se lie spécifiquement à une région fragment N-terminal du CTGF, du résidu 1 au résidu 198 de SEQ ID NO:2 ; et
(b) un deuxième anticorps qui se lie spécifiquement à une région fragment N-terminal sur le CTGF, du résidu 1 au résidu 198 de SEQ ID NO:2, différente de la région à laquelle est lié le premier anticorps.

13. Procédé selon la revendication 1 ou la revendication 2, ou trousse selon la revendication 12, dans lequel la région fragment N-terminal sur le CTGF va du résidu 23 au résidu 179 de SEQ ID NO:2.
